(19) Europäisches Patentamt
European Patent Office
Office européen des brevets

(11) Veröffentlichungsnummer : **0 119 457**
**B1**

(12) # EUROPÄISCHE PATENTSCHRIFT

(45) Veröffentlichungstag der Patentschrift : **30.12.86**

(21) Anmeldenummer : **84101457.4**

(22) Anmeldetag : **13.02.84**

(51) Int. Cl.⁴ : **C 07 C149/40**, C 07 C147/06, C 07 C147/107, C 07 C149/30, C 07 C147/14, C 07 C149/34, A 01 N 37/40, A 01 N 31/14, A 01 N 37/48, A 01 N 31/16, A 01 N 41/10

(54) **Mittel zur Regulierung des Pflanzenwachstums.**

(30) Priorität : **23.02.83 DE 3306201**
**07.12.83 DE 3344235**

(43) Veröffentlichungstag der Anmeldung : **26.09.84 Patentblatt 84/39**

(45) Bekanntmachung des Hinweises auf die Patenterteilung : **30.12.86 Patentblatt 86/52**

(84) Benannte Vertragsstaaten :
**AT BE CH DE FR GB IT LI NL SE**

(56) Entgegenhaltungen :
**EP-A- 0 056 319**
**GB-A- 771 064**
**CHEMISCHE BERICHTE, Band 111, Seiten 3497-3501, Verlag Chemie, Weinheim, DE; K. SCHANK et al.: "Synthese und Eigenschaften von Aryl-arylsulfonylmethyl-ethern"**
**SYNTHETIC COMMUNICATIONS, Band 10, Nr. 12, 1980, Seiten 911-914, Marcel Dekker Inc., New York, USA; R.A. HOLTON et al.: "A new protecting group for phenols: Phenylthiomethyl (PTM) ethers"**

(73) Patentinhaber : **BAYER AG**
**Konzernverwaltung RP Patentabteilung**
**D-5090 Leverkusen 1 Bayerwerk (DE)**

(72) Erfinder : **Schmitt, Hans-Georg, Dr.**
**Gustav-Freytag-Strasse 2**
**D-5090 Leverkusen 1 (DE)**
Erfinder : **Lürssen, Klaus, Dr.**
**August-Kierspel-Strasse 151**
**D-5060 Bergisch-Gladbach 2 (DE)**
Erfinder : **Wedemeyer, Karlfried, Dr.**
**Bilharzstrasse 7**
**D-5000 Koeln 80 (DE)**

**EP 0 119 457 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft die Verwendung von teilweise bekannten Phenoxymethan-Derivaten als Wirkstoffe zur Regulierung des Pflanzenwachstums.

Es sind bereits einige Phenoxy-methan-Derivate in der Literatur beschrieben worden (vgl. Chem. Ber. *1978*, 3497-3501). Biologische Eigenschaften dieser Verbindungen sind jedoch bisher nicht bekannt.

Aus der GB-A 771 064 sind akarizid wirksame Phenoxymethan-Derivate bekannt, in denen ein Wasserstoffatom der Methan-Gruppe durch einen substituierten Phenylthiorest und ein weiteres Wasserstoffatom der Methan-Gruppe durch einen substituierten Phenoxy-Rest ersetzt sein kann. Eine pflanzenwuchsregulierende Aktivität dieser Stoffe wird aber nicht erwähnt.

Ferner sind auch bestimmte Phenylthiomethylether bekannt, die als Schutzgruppen für Phenole verwendet werden können (vgl. Synthetic Communications *10*, 911-914 (1980)).

Es werden jedoch keine biologischen Eigenschaften dieser Stoffe offenbart.

Schließlich geht aus der EP-A 0 056 319 hervor, daß bestimmte Phenoxymethan-Derivate als Zwischenprodukte zur Herstellung von pharmakologisch wirksamen Substanzen einsetzbar sind. Wiederum werden aber keine biologischen Eigenschaften dieser Zwischenprodukte beschrieben.

Es wurde nun gefunden, daß die teilweise bekannten Phenoxy-methan-Derivate der Formel

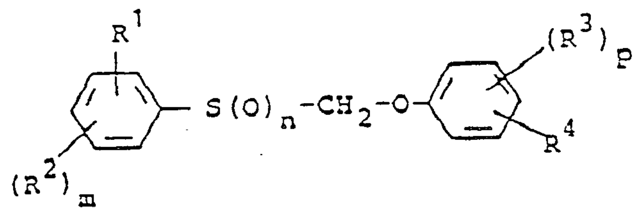

(I)

in welcher

$R^1$ für Wasserstoff, geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 5 Halogenatomen, geradkettiges oder verzweigtes Alkoxy mit 1 bis 6 Kohlenstoffatomen, Halogenalkoxy mit 1 bis 6 Kohlenstoffatomen und 1 bis 5 Halogenatomen, Alkoxycarbonyl mit 1 bis 10 Kohlenstoffatomen in der Alkoxygruppe, Cycloalkyloxycarbonyl mit 3 bis 8 Kohlenstoffatomen in der Cycloalkylgruppe, Fluor, Chlor, Brom, Jod oder Nitro steht,

$R^2$ für Wasserstoff, geradkettiges oder verzweigtes Alkyl mit 1 bis 4 Kohlenstoffatomen, Fluor, Chlor, Brom oder Jod steht,

$R^3$ für Wasserstoff, geradkettiges oder verzweigtes Alkyl mit 1 bis 6 Kohlenstoffatomen, geradkettiges oder verzweigtes Alkenyl mit 3 bis 6 Kohlenstoffatomen, geradkettiges oder verzweigtes Alkinyl mit 3 bis 6 Kohlenstoffatomen, gegebenenfalls durch Alkyl mit 1 bis 4 Kohlenstoffatomen, Fluor, Chlor, Brom und/oder Jod substituiertes Phenyl, gegebenenfalls durch Alkyl mit 1 bis 4 Kohlenstoffatomen, Fluor, Chlor, Brom und/oder Jod substituiertes Benzyl, Alkoxy mit 1 bis 6 Kohlenstoffatomen, Phenoxy, Halogenalkyl mit 1 bis 6 Kohlenstoffatomen und 1 bis 5 Halogenatomen, Halogenalkoxy mit 1 bis 6 Kohlenstoffatomen und 1 bis 5 Halogenatomen, Alkylthio mit 1 bis 6 Kohlenstoffatomen, Halogenalkylthio mit 1 bis 6 Kohlenstoffatomen und 1 bis 5 Halogenatomen, Alkylsulfonyl mit 1 bis 6 Kohlenstoffatomen, Phenylthio, Phenylsulfonyl, Nitro, Fluor, Chlor, Brom oder Jod steht,

$R^4$ für Wasserstoff, geradkettiges oder verzweigtes Alkyl mit 1 bis 6 Kohlenstoffatomen, Fluor, Chlor, Brom, Jod, Cycloalkyloxycarbonyl mit bis zu 10 Kohlenstoffatomen in der Cycloalkylgruppe, Aminocarbonyl, Alkylaminocarbonyl mit 1 bis 6 Kohlenstoffatomen in der Alkylgruppe, Dialkylaminocarbonyl mit 1 bis 6 Kohlenstoffatomen in jeder Alkylgruppe, Cycloalkylaminocarbonyl mit 3 bis 8 Kohlenstoffatomen in der Cycloalkylgruppe, Dicycloalkyl-aminocarbonyl mit 3 bis 8 Kohlenstoffatomen in jeder Cycloalkylgruppe, gegebenenfalls durch Alkyl mit 1 bis 4 Kohlenstoffatomen, Fluor, Chlor, Brom und/oder Jod substituiertes Phenoxycarbonyl, für Cyano oder den Rest der Formel —COOR steht, worin

R für Wasserstoff, ein Natrium- oder Kalium-Kation, ein Äquivalent eines Magnesium- oder Calcium-Kations, für Alkyl mit 1 bis 10 Kohlenstoffatomen, Alkenyl mit 2 bis 10 Kohlenstoffatomen, Alkoxyalkyl mit 1 bis 6 Kohlenstoffatomen in der Alkoxygruppe und 1 bis 6 Kohlenstoffatomen im Alkylteil, Alkylthioalkyl mit 1 bis 6 Kohlenstoffatomen in der Alkylthiogruppe und 1 bis 6 Kohlenstoffatomen im Alkylteil oder für den Rest der Formel $—CH_2—O—(CH_2—CH_2—O)_q$-Alk steht, worin

Alk für Alkyl mit 1 bis 6 Kohlenstoffatomen steht und

q für 0, 1 oder 2 steht,

m für 1 oder 2 steht,

n für 0, 1 oder 2 steht und

p für 1 oder 2 steht,

sehr gut zur Regulierung des Pflanzenwachstums geeignet sind.

Die erfindungsgemäß verwendbaren Stoffe sind durch die Formel (I) allgemein definiert.

Bevorzugt sind diejenigen Stoffe der Formel (I), in denen

$R^1$ für Wasserstoff, geradkettiges oder verzweigtes Alkyl mit 1 bis 4 Kohlenstoffatomen, geradkettiges oder verzweigtes Alkoxy mit 1 bis 4 Kohlenstoffatomen, Halogenalkoxy mit 1 bis 3 Kohlenstoffatomen und 1 bis 5 Fluor-, Chlor- und/oder Brom-Atomen, Halogenalkyl mit 1 bis 3 Kohlenstoffatomen und 1 bis 5 Fluor-, Chlor- und/oder Brom-Atomen, Alkoxycarbonyl mit 1 bis 4 Kohlenstoffatomen in der Alkoxygruppe, Cycloalkyloxycarbonyl mit 3 bis 6 Kohlenstoffatomen in der Cycloalkylgruppe, Fluor, Chlor, Brom oder Nitro steht,

$R^2$ für Wasserstoff, geradkettiges oder verzweigtes Alkyl mit 1 bis 4 Kohlenstoffatomen, Fluor, Chlor oder Brom steht,

$R^3$ für Wasserstoff, geradkettiges oder verzweigtes Alkyl mit 1 bis 4 Kohlenstoffatomen, geradkettiges oder verzweigtes Alkenyl mit 3 bis 5 Kohlenstoffatomen, geradkettiges oder verzweigtes Alkinyl mit 3 bis 5 Kohlenstoffatomen, gegebenenfalls durch Alkyl mit 1 bis 3 Kohlenstoffatomen, Fluor, Chlor und/oder Brom substituiertes Phenyl, gegebenenfalls durch Alkyl mit 1 bis 3 Kohlenstoffatomen, Fluor, Chlor und/oder Brom substituiertes Benzyl, Alkoxy mit 1 bis 4 Kohlenstoffatomen, Phenoxy, Halogenalkyl mit 1 bis 3 Kohlenstoffatomen und 1 bis 5 Fluor-, Chlor- und/oder Brom-Atomen, Halogenalkoxy mit 1 bis 3 Kohlenstoffatomen und 1 bis 5 Fluor-, Chlor- und/oder Brom-Atomen, Alkylthio mit 1 bis 4 Kohlenstoffatomen, Halogenalkylthio mit 1 bis 3 Kohlenstoffatomen und 1 bis 5 Fluor-, Chlor- und/oder Brom-Atomen, Alkylsulfonyl mit 1 bis 4 Kohlenstoffatomen, Phenylthio, Phenylsulfonyl, Nitro, Fluor, Chlor oder Brom steht,

$R^4$ für Wasserstoff, geradkettiges oder verzweigtes Alkyl mit 1 bis 4 Kohlenstoffatomen, Fluor, Chlor, Brom, Cycloalkyloxycarbonyl mit 3 bis 8 Kohlenstoffatomen in der Cycloalkylgruppe, Aminocarbonyl, Alkylaminocarbonyl mit 1 bis 4 Kohlenstoffatomen in der Alkylgruppe, Dialkylaminocarbonyl mit 1 bis 4 Kohlenstoffatomen in jeder Alkylgruppe, Cycloalkylaminocarbonyl mit 3 bis 7 Kohlenstoffatomen in der Cycloalkylgruppe, Dicycloalkyl-aminocarbonyl mit 3 bis 7 Kohlenstoffatomen in jeder Cycloalkylgruppe, gegebenenfalls durch Alkyl mit 1 bis 3 Kohlenstoffatomen, Fluor, Chlor und/oder Brom substituiertes Phenoxycarbonyl, für Cyano oder für den Rest der Formel —COOR steht, worin

R für Wasserstoff, ein Natrium- oder Kaliumkation, ein Äquivalent eines Magnesium- oder Calium-Kations, Alkyl mit 1 bis 8 Kohlenstoffatomen, Alkenyl mit 2 bis 8 Kohlenstoffatomen, Alkoxyalkyl mit 1 bis 4 Kohlenstoffatomen in der Alkoxygruppe und 1 bis 4 Kohlenstoffatomen im Alkylteil, Alkylthioalkyl mit 1 bis 4 Kohlenstoffatomen in der Alkylthiogruppe und 1 bis 4 Kohlenstoffatomen im Alkylteil oder für den Rest der Formel $—CH_2—O—(CH_2—CH_2—O)_q$-Alk steht, worin

Alk für Alkyl mit 1 bis 4 Kohlenstoffatomen steht und

q für 0, 1 oder 2 steht,

m für 1 oder 2 steht,

n für 0, 1 oder 2 steht und

p für 1 oder 2 steht.

Eine besonders bevorzugte Gruppe von erfindungsgemäß verwendbaren Verbindungen sind die Stoffe der Formel

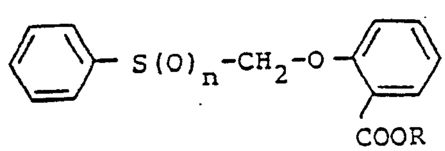

(Ia)

in welcher

n für 0, 1 oder 2 steht und

R für Wasserstoff, ein Natrium- oder Kalium-Kation, ein Äquivalent eines Magnesium- oder Calcium-Kations, Alkyl mit 1 bis 8 Kohlenstoffatomen, Alkenyl mit 2 bis 8 Kohlenstoffatomen, Alkoxyalkyl mit 1 bis 4 Kohlenstoffatomen in der Alkoxygruppe und 1 bis 4 Kohlenstoffatomen im Alkylteil, Alkylthioalkyl mit 1 bis 4 Kohlenstoffatomen in der Alkylthiogruppe und 1 bis 4 Kohlenstoffatomen im Alkylteil oder für den Rest der Formel $—CH_2—O—(CH_2—CH_2—O)_q$-Alk steht, worin

Alk für Alkyl mit 1 bis 4 Kohlenstoffatomen steht und

q für 0, 1 oder 2 steht.

Als Beispiele für erfindungsgemäß verwendbare Phenoxy-methan-Derivate der Formel (I) seien genannt :

1-Phenoxy-1-phenylthio-methan
1-Phenoxy-1-phenylsulfinyl-methan
1-Phenoxy-1-phenylsulfonyl-methan
1-(4,6-Dibrom-2-methoxycarbonyl-phenoxy)-1-phenylthio-methan
1-(5-Chlor-2-methoxycarbonyl-phenoxy)-1-phenylthio-methan
1-(4-Brom-2-ethoxycarbonyl-phenoxy)-1-phenylthio-methan
1-(2-Ethoxycarbonyl-4-fluor-phenoxy)-1-phenylthio-methan
1-(6-Methoxy-2-methoxycarbonyl-phenoxy)-1-phenylthio-methan

1-(4-Trifluormethyl-phenoxy)-1-phenylsulfonyl-methan
1-[2-(3-Methyl-phenyl)-oxycarbonyl-phenoxy]-1-phenylthio-methan
1-[2-(4-Chlor-phenyl)-oxycarbonyl-phenoxy]-1-phenylthio-methan
1-(3-Phenoxy-phenoxy)-1-phenylthio-methan
1-(3-Trifluormethoxy-phenoxy)-1-phenylthio-methan
1-(4-Trifluormethylthio-phenoxy)-1-phenylthio-methan
1-(2-Chlor-4-trifluormethyl-phenoxy)-1-phenylthio-methan
1-(2-Isopropoxycarbonyl-6-nitro-phenoxy)-1-phenylthio-methan
1-(2-Propoxycarbonyl-4-nitro-phenoxy)-1-phenylthio-methan
1-(2-Ethoxycarbonyl-4,6-dinitro-phenoxy)-1-phenylthio-methan
1-(2-Methoxy-6-methyl-phenoxy)-1-phenylthio-methan
1-(4-Chlor-2-ethoxycarbonyl-6-methyl)-1-phenylthio-methan
1-(2-Methoxycarbonyl-4-methyl-phenoxy)-1-phenylthio-methan
1-(2-Ethoxycarbonyl-5-methyl-phenoxy)-1-phenylthio-methan
1-(2-Ethoxycarbonyl-4-tert.-butyl-phenoxy)-1-phenylthio-methan
1-(2-Methoxycarbonyl-phenoxy)-1-phenylthio-methan
1-(2-Methoxycarbonyl-phenoxy)-1-phenylsulfinyl-methan
1-(2-Methoxycarbonyl-phenoxy)-1-phenylsulfonyl-methan
1-(2-Methoxycarbonyl-6-trifluormethoxy-phenoxy)-1-phenylthio-methan
1-(2-Methoxycarbonyl-4-trifluormethyl-phenoxy)-1-phenylthio-methan
1-(2-Ethoxycarbonyl-phenoxy)-1-phenylthio-methan
1-(2-Ethoxycarbonyl-phenoxy)-1-phenylsulfinyl-methan
1-(2-Ethoxycarbonyl-phenoxy)-1-phenylsulfonyl-methan
1-(2-Methoxycarbonyl-phenoxy)-1-(2-trifluormethylphenylthio)-methan
1-(2-Methoxycarbonyl-phenoxy)-1-(2-nitro-phenylthio)-methan
1-(2-Ethoxycarbonyl-phenoxy)-1-(2-nitro-phenylthio)-methan
1-(2-Methoxycarbonyl-phenoxy)-1-(2,3-dimethyl-phenylthio)-methan
1-(2-Propoxycarbonyl-phenoxy)-1-phenylthio-methan
1-(2-Propoxycarbonyl-phenoxy)-1-phenylsulfinyl-methan
1-(2-Propoxycarbonyl-phenoxy)-1-phenylsulfonyl-methan
1-(2-Isopropoxycarbonyl-phenoxy)-1-phenylthio-methan
1-(2-Isopropoxycarbonyl-phenoxy)-1-phenylsulfinyl-methan
1-(2-Isopropoxycarbonyl-phenoxy)-1-phenylsulfonyl-methan
1-(2-Butyloxycarbonyl-phenoxy)-1-phenylthio-methan
1-(2-Butyloxycarbonyl-phenoxy)-1-phenylsulfinyl-methan
1-(2-Butyloxycarbonyl-phenoxy)-1-phenylsulfonyl-methan
1-(2-Isobutyloxycarbonyl-phenoxy)-1-phenylthio-methan
1-(2-sek.-Butyloxycarbonyl-phenoxy)-1-phenylthio-methan
1-(2-Pentyloxycarbonyl-phenoxy)-1-phenylthio-methan
1-(2-Hexyloxycarbonyl-phenoxy)-1-phenylthio-methan
1-(2-Heptyloxycarbonyl-phenoxy)-1-phenylthio-methan
1-(2-Octyloxycarbonyl-phenoxy)-1-phenylthio-methan
1-(2-Cyclohexyloxycarbonyl-phenoxy)-1-phenylthio-methan
1-Phenoxy-1-(4-chlorphenylthio)-methan
1-Phenoxy-1-(4-tolylthio)-methan
1-(2-Phenylphenoxy)-1-(4-chlorphenylthio)-methan
1-Phenoxy-1-(4-chlorphenylsulfonyl)-methan
1-Phenoxy-1-(4-tolylsulfonyl)-methan
1-(2-Phenyl-phenoxy)-1-(4-chlorphenylsulfonyl)-methan
1-(4-Chlor-3-methyl-phenoxy)-1-(4-chlorphenylthio)-methan
1-(4-Chlor-3-methyl-phenoxy)-1-(4-tolylthio)-methan
1-(4-Chlor-3-methyl-phenoxy)-1-phenylthio-methan
1-(4-Chlor-phenoxy)-1-(4-chlorphenylthio)-methan
1-(4-Chlor-phenoxy)-1-(4-chlorphenylsulfinyl)-methan
1-(4-Chlor-phenoxy)-1-(4-chlorphenylsulfonyl)-methan
1-(4-Chlor-phenoxy)-1-(4-tolylthio)-methan
1-(4-Chlor-phenoxy)-1-phenylthio-methan
1-(4-Chlor-3-methyl-phenoxy)-1-(4-chlorphenylsulfonyl)-methan
1-(4-Chlor-3-methyl-phenoxy)-1-(4-tolylsulfonyl)-methan
1-(4-Chlor-3-methyl-phenoxy)-1-phenylsulfinyl-methan
1-(4-Chlor-phenoxy)-1-(4-tolylsulfonyl)-methan
1-(4-Chlor-phenoxy)-1-phenylsulfonyl-methan
1-(2-Phenyl-phenoxy)-1-phenylthio-methan
1-(2-Phenyl-phenoxy)-1-(4-tolylthio)-methan
1-(2-Phenyl-phenoxy)-1-phenylsulfonyl-methan

1-(2-Phenyl-phenoxy)-1-(4-tolylsulfonyl)-methan
1-Phenoxy-1-(4-tert.-butyl-phenylthio)-methan
1-(4-Chlorphenoxy)-1-(4-tert.-butyl-phenylthio)-methan
1-Phenoxy-1-(4-tert.-butyl-phenylsulfonyl)-methan
1-(4-Chlor-3-methyl-phenoxy)-1-(4-tert.-butyl-phenylsulfinyl)-methan
1-(2-Isopropoxy-phenoxy)-1-(4-chlorphenylthio)-methan
1-(2-Methoxycarbonyl-phenoxy)-1-(4-chlor-phenylthio)-methan
1-(3-Methyl-phenoxy)-1-(4-chlor-phenylthio)-methan
1-(2,4-Dichlor-phenoxy)-1-(4-tolylthio)-methan
1-(2-Isopropoxy-phenoxy)-1-(4-tolylthio)-methan
1-(2-Methoxycarbonyl-phenoxy)-1-(4-chlor-phenylsulfonyl)-methan
1-(2-Isopropoxy-phenoxy)-1-(4-chlorphenylsulfonyl)-methan
1-(3-Methyl-phenoxy)-1-(4-tolylsulfonyl)-methan
1-(2,4-Dichlor-phenoxy)-1-(4-tolylsulfonyl)-methan
1-(2-Methoxycarbonyl-phenoxy)-1-(4-tolylthio)-methan
1-(3-Methoxy-phenoxy)-1-(4-chlorphenylthio)-methan
1-(2-Methoxycarbonyl-phenoxy)-1-(4-tolylsulfonyl)-methan
1-(2-Methoxycarbonyl-phenoxy)-1-phenylsulfonyl-methan
1-(2,4-Dichlor-phenoxy)-1-(2-methoxycarbonyl-phenylthio)-methan
1-(2,4-Dichlor-phenoxy)-1-(2-methoxycarbonyl-phenylsulfinyl)-methan
1-(2,4-Dichlor-phenoxy)-1-(2-methoxycarbonyl-phenylsulfonyl)-methan
1-(2-Methoxycarbonyl-phenoxy)-1-(3,4-dichlor-phenylthio)-methan
1-(2-Methoxycarbonyl-phenoxy)-1-(2,5-dichlor-phenylthio)-methan
1-(2-Methoxycarbonyl-phenoxy)-1-(4-tert.-butyl-phenylthio)-methan
1-[2-(3-Methyl-butyl)-oxycarbonyl-phenoxy]-1-phenylthio-methan
1-(2-Phenoxycarbonyl-phenoxy)-1-(phenylthio)-methan
1-(2-Aminocarbonyl-phenoxy)-1-phenylthio-methan
1-(3-Methoxycarbonyl-phenoxy)-1-phenylthio-methan
1-(3-Methoxycarbonyl-phenoxy)-1-phenylsulfinyl-methan
1-(3-Methoxycarbonyl-phenoxy)-1-phenylsulfonyl-methan
1-(4-Ethoxycarbonyl-phenoxy)-1-phenylthio-methan
1-(4-Propoxycarbonyl-phenoxy)-1-phenylthio-methan
1-(2-Cyano-4-nitro-phenoxy)-1-phenylthio-methan
1-(4-Cyano-phenoxy)-1-phenylthio-methan
1-(2,6-Dibrom-4-cyano-phenoxy)-1-phenylthio-methan
1-(2,6-Dibrom-4-cyano-phenoxy)-1-phenylsulfinyl-methan
1-(2,6-Dibrom-4-cyano-phenoxy)-1-phenylsulfonyl-methan

Die erfindungsgemäß verwendbaren Phenoxymethan-Derivate sind teilweise bekannt (vgl. Chem. Ber. *1978*, 3497-3501). Die erfindungsgemäß verwendbaren Phenoxy-methan-Derivate der Formel

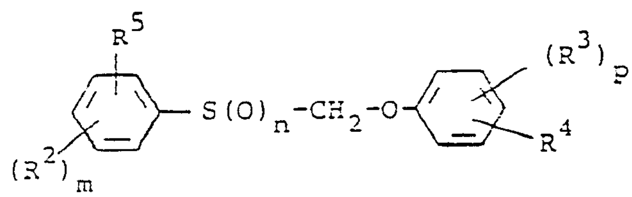

(Ib)

in welcher

$R^5$ für geradkettiges oder verzweigtes Alkyl mit 2 bis 4 Kohlenstoffatomen, geradkettiges oder verzweigtes Alkoxy mit 1 bis 4 Kohlenstoffatomen, Halogenalkyl mit 1 bis 3 Kohlenstoffatomen und 1 bis 5 Fluor-, Chlor- und/oder Brom-Atomen, Halogenalkoxy mit 1 bis 3 Kohlenstoffatomen und 1 bis 5 Fluor-, Chlor- und/oder Brom-Atomen, Alkoxycarbonyl mit 1 bis 4 Kohlenstoffatomen in der Alkoxygruppe, Cycloalkyloxycarbonyl mit 3 bis 6 Kohlenstoffatomen in der Cycloalkylgruppe, Fluor, Brom oder Nitro steht,

$R^2$ für Wasserstoff, geradkettiges oder verzweigtes Alkyl mit 1 bis 4 Kohlenstoffatomen, Fluor, Chlor oder Brom steht,

$R^3$ für Wasserstoff, geradkettiges oder verzweigtes Alkyl mit 1 bis 4 Kohlenstoffatomen, geradkettiges oder verzweigtes Alkenyl mit 3 bis 5 Kohlenstoffatomen, geradkettiges oder verzweigtes Alkinyl mit 3 bis 5 Kohlenstoffatomen, gegebenenfalls durch Alkyl mit 1 bis 3 Kohlenstoffatomen, Fluor, Chlor und/oder Brom substituiertes Phenyl, gegebenenfalls durch Alkyl mit 1 bis 3 Kohlenstoffatomen, Fluor, Chlor und/oder Brom substituiertes Benzyl, Alkoxy mit 1 bis 4 Kohlenstoffatomen, Phenoxy, Halogenalkyl mit 1 bis 3 Kohlenstoffatomen und 1 bis 5 Fluor-, Chlor- und/oder Brom-Atomen, Halogenalkoxy mit 1 bis 3 Kohlenstoffatomen und 1 bis 5 Fluor-, Chlor- und/oder Brom-Atomen, Alkylthio mit 1 bis 4 Kohlenstoffatomen, Halogenalkylthio mit 1 bis 3 Kohlenstoffatomen und 1 bis 5 Fluor-, Chlor- und/oder Brom-Atomen, Alkylsulfonyl mit 1 bis 4 Kohlenstoffatomen, Phenylthio, Phenylsulfonyl, Nitro, Fluor, Chlor oder Brom steht,

$R^4$ für Wasserstoff, geradkettiges oder verzweigtes Alkyl mit 1 bis 4 Kohlenstoffatomen, Fluor, Chlor, Brom, Alkoxycarbonyl mit 1 bis 8 Kohlenstoffatomen in der Alkoxygruppe, Cycloalkyloxycarbonyl mit 3 bis 8 Kohlenstoffatomen in der Cycloalkylgruppe, Aminocarbonyl, Alkylaminocarbonyl mit 1 bis 4 Kohlenstoffatomen in der Alkylgruppe, Dialkylaminocarbonyl mit 1 bis 4 Kohlenstoffatomen in jeder Alkylgruppe, Cycloalkylaminocarbonyl mit 3 bis 7 Kohlenstoffatomen in der Cycloalkylgruppe, Dicycloalkyl-aminocarbonyl mit 3 bis 7 Kohlenstoffatomen in jeder Cycloalkylgruppe, gegebenenfalls durch Alkyl mit 1 bis 3 Kohlenstoffatomen, Fluor, Chlor und/oder Brom substituiertes Phenoxycarbonyl oder für Cyano steht,

m für 1 oder 2 steht,

n für 1 oder 2 steht und

p für 1 oder 2 steht, und

$R^5$ außerdem auch für Wasserstoff oder Chlor steht, sofern $R^3$ nicht für Wasserstoff, Chlor, Methyl oder Nitro steht,

sind bisher noch nicht bekannt.

Die Phenoxy-methan-Derivate der Formel (Ib) lassen sich nach mehreren Verfahren in einfacher Weise herstellen. So erhält man Phenoxy-methan-Derivate der Formel (Ib), indem man

a) Thiophenol-Derivate der Formel

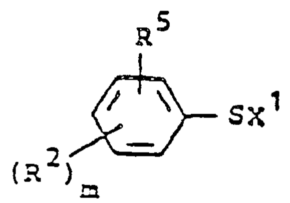

(II)

in welcher

$R^2$, $R^5$ und m die oben angegebene Bedeutung haben und

$X^1$ für Wasserstoff oder ein Alkalimetallatom steht,

mit Halogenmethyl-phenyl-ethern der Formel

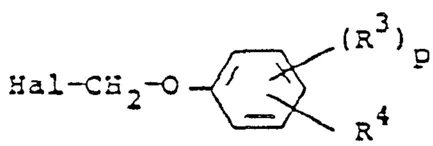

(III)

in welcher

$R^3$, $R^4$ und p die oben angegebene Bedeutung haben und

Hal für Chlor, Brom oder Jod steht,

gegebenenfalls in Gegenwart eines Säureakzeptors sowie gegebenenfalls in Gegenwart eines Verdünnungsmittels umsetzt und anschließend die dabei entstehenden Phenoxymethan-Derivate der Formel

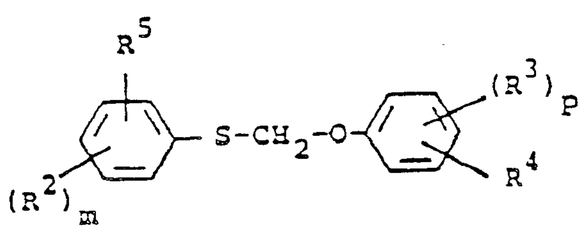

(Ic)

in welcher $R^2$, $R^3$, $R^4$, $R^5$, m und p die oben angegebene Bedeutung haben, mit einem Oxidationsmittel gegebenenfalls in Gegenwart eines Verdünnungsmittels umsetzt, oder

b) Phenol-Derivate der Formel

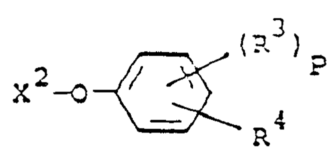

(IV)

in welcher
$R^3$, $R^4$ und p die oben angegebene Bedeutung haben und
$X^2$ für Wasserstoff oder ein Alkalimetallatom steht,
mit Halogenmethyl-phenylsulfiden der Formel

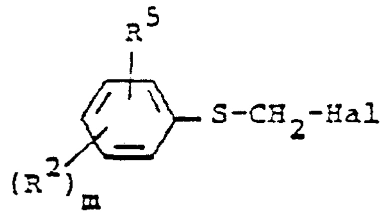

(V)

in welcher
$R^2$, $R^5$ und m die oben angegebene Bedeutung haben und
Hal für Chlor, Brom oder Jod steht,
gegebenenfalls in Gegenwart eines Säureakzeptors sowie gegebenenfalls in Gegenwart eines Verdünnungsmittels umsetzt und anschließend die dabei entstehenden Phenoxymethan-Derivate der Formel

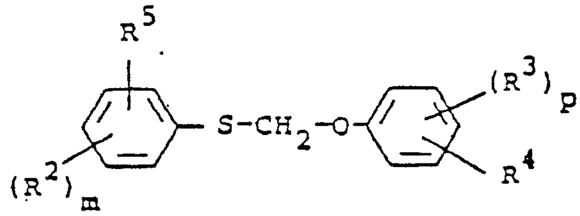

(Ic)

in welcher $R^2$, $R^3$, $R^4$, $R^5$, m und p die oben angegebene Bedeutung haben, mit einem Oxidationsmittel gegebenenfalls in Gegenwart eines Verdünnungsmittels umsetzt, oder

c) Sulfinsäure-Salze der Formel

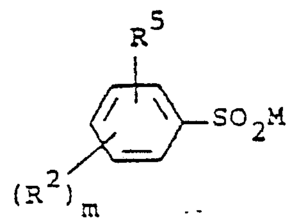

(VI)

in welcher
$R^2$, $R^5$ und m die oben angegebene Bedeutung haben und
M für ein Alkalimetallatom steht,
mit Halogenmethyl-phenyl-ethern der Formel

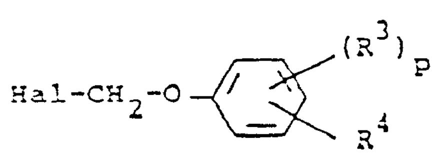

(III)

in welcher
$R^3$, $R^4$ und p die oben angegebene Bedeutung haben und
Hal für Chlor, Brom oder Jod steht,
gegebenenfalls in Gegenwart eines Katalysators sowie gegebenenfalls in Gegenwart eines Verdünnungsmittels umsetzt.

Ebenfalls noch nicht bekannt sind die erfindungsgemäß verwendbaren Phenoxy-methan-Derivate der Formel

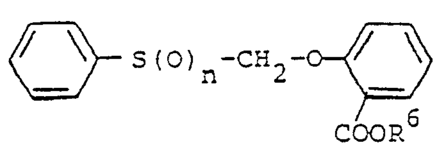

(Id)

in welcher

n für 0, 1 oder 2 steht und

$R^6$ für Wasserstoff, ein Natrium- oder Kalium-Kation, ein Äquivalent eines Magnesium- oder Calcium-Kations, Alkyl mit 2 bis 8 Kohlenstoffatomen, Alkenyl mit 2 bis 8 Kohlenstoffatomen, Alkoxyalkyl mit 1 bis 4 Kohlenstoffatomen in der Alkoxygruppe und 1 bis 4 Kohlenstoffatomen im Alkylteil, Alkylthioalkyl mit 1 bis 4 Kohlenstoffatomen in der Alkylthiogruppe und 1 bis 4 Kohlenstoffatomen im Alkylteil oder für den Rest der Formel $-CH_2-O-(CH_2-CH_2-O)_q$-Alk steht, worin

Alk für Alkyl mit 1 bis 4 Kohlenstoffatomen steht und

q für 0, 1 oder 2 steht.

Die Phenoxy-methan-Derivate der Formel (Id) lassen sich herstellen, indem man

d) Verbindungen der Formel

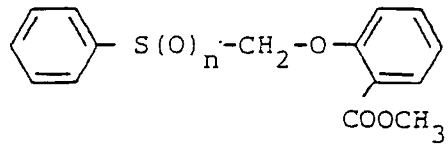

(Ie)

in welcher n die oben angegebene Bedeutung hat, entweder

α) mit Verbindungen der Formel

$R^7OH$ (VII)

in welcher $R^7$ für Alkyl mit 2 bis 8 Kohlenstoffatomen, Alkenyl mit 2 bis 8 Kohlenstoffatomen, Alkoxyalkyl mit 1 bis 4 Kohlenstoffatomen in der Alkoxygruppe und 1 bis 4 Kohlenstoffatomen im Alkylteil, Alkylthioalkyl mit 1 bis 4 Kohlenstoffatomen in der Alkylthiogruppe und 1 bis 4 Kohlenstoffatomen im Alkylteil oder für den Rest der Formel $-CH_2-O-(CH_2-CH_2-O)_q$-Alk steht, worin

Alk für Alkyl mit 1 bis 4 Kohlenstoffatomen steht und

q für 0, 1 oder 2 steht,

in Gegenwart einer Base sowie gegebenenfalls

in Gegenwart eines Verdünnungsmittels umsetzt, oder

β) mit Basen der Formel

$M^1OH$ (VIII)

in welcher $M^1$ für ein Natrium- oder Kaliumkation oder für ein Äquivalent eines Magnesium- oder Calciumkations steht, in Gegenwart eines Verdünnungsmittels umsetzt und dann mit einer starken Säure ansäuert und gegebenenfalls anschließend erneut mit einer Base der Formel

$M^1OH$ (VIII)

in welcher $M^1$ die oben angegebene Bedeutung hat, in Gegenwart eines Verdünnungsmittels umsetzt.

Diejenigen Stoffe der Formel (I), die nicht von den Formeln (Ib) und (Id) umfaßt werden, lassen sich ebenfalls nach den oben angegebenen Verfahren herstellen.

Verwendet man 2-Mercapto-benzoesäure-methylester und Chlormethyl-(2,4-dichlor-phenyl)-ether als Ausgangsstoffe, so läßt sich der Verlauf des erfindungsgemäßen Verfahrens (a) durch das folgende Formelschema wiedergeben :

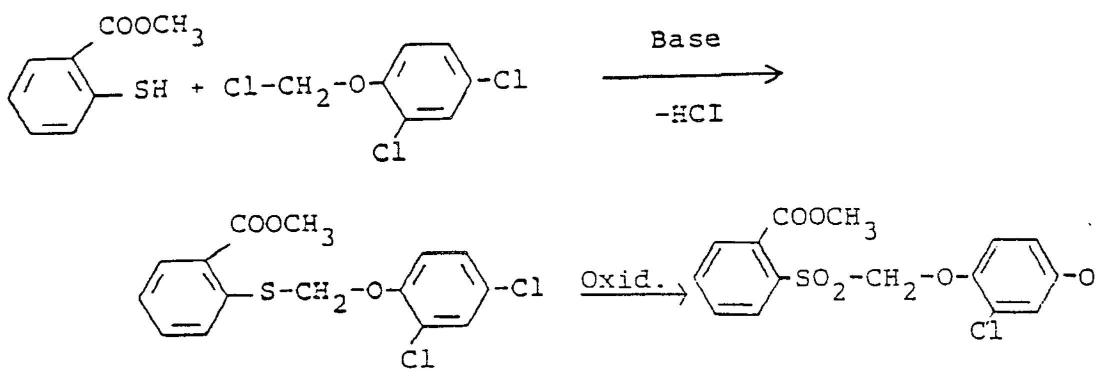


Verwendet man Natriumphenolat und Chlormethyl-phenyl-sulfid als Ausgangsstoffe, so kann der

Verlauf des erfindungsgemäßen Verfahrens (b) durch das folgende Formelschema wiedergegeben werden :

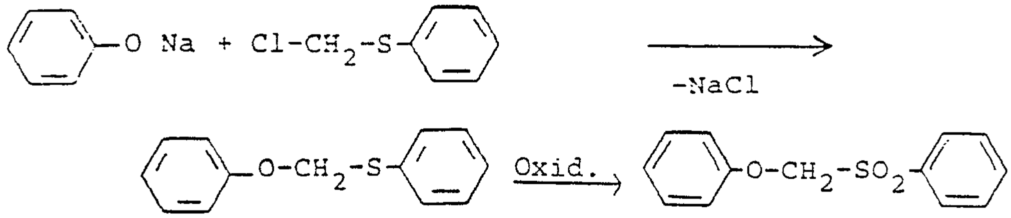


Verwendet man Natrium-(4-chlor-benzol)-sulfinat und Chlormethyl-(4-ethyl-phenyl)-ether als Ausgangsstoffe, so kann der Verlauf des erfindungsgemäßen Verfahrens (c) durch das folgende Formelschema wiedergegeben werden :

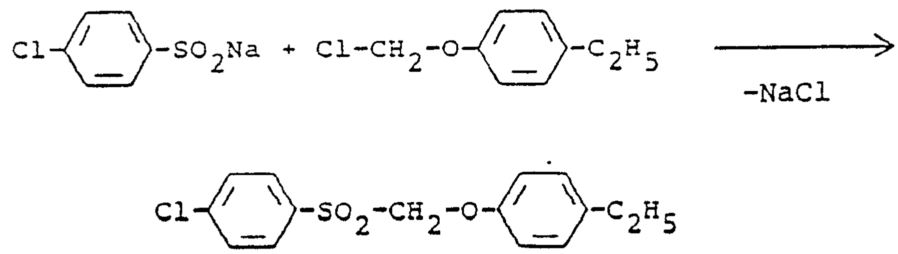


Verwendet man 2-(Phenylthio-methoxy)-benzoesäure-methylester und n-Octanol als Ausgangsstoffe, so kann der Verlauf des erfindungsgemäßen Verfahrens (d, Variante α) durch das folgende Formelschema wiedergegeben werden :

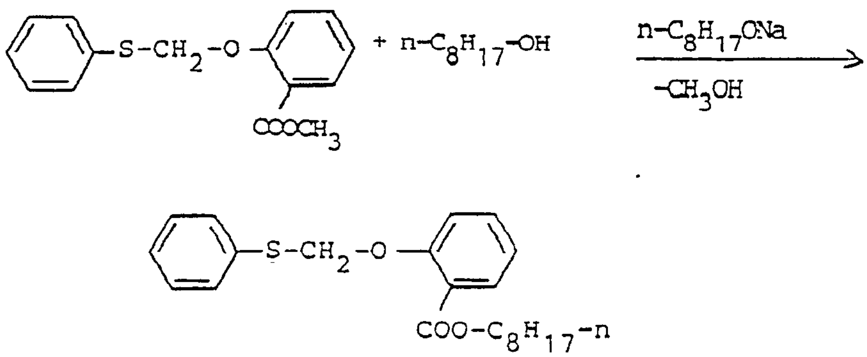


Verwendet man 2-(Phenylthio-methoxy)-benzoesäure-methylester als Ausgangsstoff, Kaliumhydroxid als Base und Salzsäure zum Acidifizieren, so kann der Verlauf des erfindungsgemäßen Verfahrens (d, Variante β) durch das folgende Formelschema wiedergegeben werden :

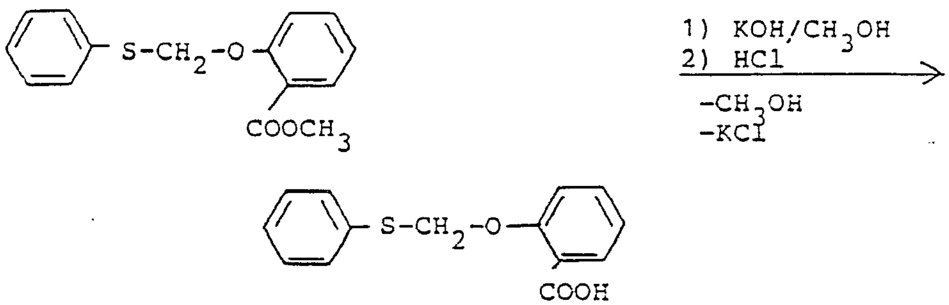


Die bei dem erfindungsgemäßen Verfahren (a) als Ausgangsstoffe benötigten Thiophenol-Derivate sind durch die Formel (II) allgemein definiert. In dieser Formel haben $R^2$, $R^5$ und m diejenigen Bedeutungen, die bereits im Zusammenhang mit der Beschreibung der Verbindungen der Formel (Ib) für diese Reste bzw. für diesen Index genannt wurden. $X^1$ steht vorzugsweise für Wasserstoff, Natrium oder Kalium.

Die Thiophenol-Derivate der Formel (II) sind bekannt oder lassen sich nach bekannten Verfahren in einfacher Weise herstellen (vgl. Houben-Weyl « Methoden der Organischen Chemie », Bd. IX, Thieme-Verlag, Stuttgart 1955).

Die bei dem erfindungsgemäßen Verfahren (a) weiterhin als Ausgangsstoffe benötigten Halogenmethyl-phenyl-ether sind durch die Formel (III) allgemein definiert.

In dieser Formel haben $R^3$, $R^4$ und p diejenigen Bedeutungen, die bereits im Zusammenhang mit der Beschreibung der Verbindungen der Formel (Ib) für diese Reste bzw. für diesen Index genannt wurden. Hal steht vorzugsweise für Chlor oder Brom.

Die Halogenmethyl-phenyl-ether der Formel (III) sind bekannt oder lassen sich nach bekannten Methoden in einfacher Weise herstellen (vgl. J. Appl. Chem. *3*, (1953), 252-263).

Als Säureakzeptoren können bei der Durchführung des erfindungsgemäßen Verfahrens (a) alle üblicherweise für derartige Umsetzungen geeigneten Säurebindemittel verwendet werden. Vorzugsweise in Frage kommen Alkali- und Erdalkalicarbonate, wie Natriumcarbonat, Kaliumcarbonat, Magnesiumcarbonat und Calciumcarbonat, ferner Alkali-alkoholate, wie Natrium- und Kaliummethylat bzw. -ethylat, ferner aliphatische, aromatische oder heterocyclische Amine, beispielsweise Triethylamin, Trimethylamin, Dimethylanilin, Dimethylbenzyl-amin, 1,5-Diazabicyclo-[4,3,0]-non-5-en (DBN), 1,8-Diazabicyclo-[5,4,0]-undec-7-en (DBU) und Pyridin.

Als Verdünnungsmittel kommen bei der Durchführung des erfindungsgemäßen Verfahrens (a) praktisch alle inerten organischen Lösungsmittel in Betracht. Vorzugsweise verwendbar sind aliphatische und aromatische, gegebenenfalls halogenierte Kohlenwasserstoffe wie Pentan, Hexan, Heptan, Cyclohexan ; Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glykoldimethylether und Diglykoldimethylether, Tetrahydrofuran und Dioxan, Alkohole, wie Methanol und Ethanol, ferner Ester, wie Essigsäure-methylester und -ethylester, Nitrile, wie z. B. Acetonitril und Propionitril, Amide, wie z. B. Dimethylformamid, Dimethylacetamid und N-Methylpyrrolidon, sowie Dimethylsulfoxid, Tetramethylensulfon und Hexamethylphosphorsäuretriamid.

Die Reaktionstemperaturen können bei dem erfindungsgemäßen Verfahren (a) innerhalb eines größeren Bereiches variiert werden. Im allgemeinen arbeitet man bei Temperaturen zwischen 0 °C und 100 °C, vorzugsweise zwischen 20 °C und 80 °C.

Das erfindungsgemäße Verfahren (a) wird im allgemeinen bei Normaldruck durchgeführt. Es ist jedoch auch möglich, unter erhöhtem oder vermindertem Druck zu arbeiten.

Zur Durchführung des erfindungsgemäßen Verfahrens (a) werden die Ausgangsstoffe der Formeln (II) und (III) im allgemeinen in etwa äquimolaren Mengen eingesetzt. Es ist jedoch auch möglich, eine der beiden Komponenten in einem größeren Überschuß einzusetzen. Die Reaktionen werden im allgemeinen in einem geeigneten Verdünnungsmittel, gegebenenfalls in Gegenwart eines Säurebindemittels, durchgeführt und das Reaktionsgemisch wird bis zur Beendigung der Umsetzung bei der jeweils erforderlichen Temperatur nachgerührt. Die Aufarbeitung erfolgt jeweils nach üblichen Methoden.

Zur Herstellung derjenigen Verbindungen der Formel (Ib), in denen n für 1 oder 2 steht, werden die bei dem erfindungsgemäßen Verfahren (a) zunächst entstehenden Phenoxymethan-Derivate der Formel (Ic) mit einem Oxidationsmittel, gegebenenfalls in Gegenwart eines Verdünnungsmittels, umgesetzt.

Als Oxidationsmittel können dabei alle üblichen sauerstoffabgebenden Oxidationsmittel eingesetzt werden, die in der Lage sind, Sulfide in Sulfoxide bzw. in Sulfone zu überführen. Vorzugsweise in Frage kommen Wasserstoffperoxid, Peressigsäure und m-Chlorperbenzoesäure.

Als Verdünnungsmittel kommen bei der Durchführung dieser Oxidation alle üblicherweise für derartige Oxidationen verwendbaren organischen Solventien in Betracht. Vorzugsweise verwendbar sind Alkohole, wie Methanol oder Ethanol, ferner Carbonsäuren, wie Ameisensäure und Essigsäure. Außerdem kann auch Wasser gegebenenfalls im Gemisch mit einem der genannten organischen Lösungsmittel eingesetzt werden. Die Oxidation mit m-Chlorperbenzoesäure wird bevorzugt in halogenierten Kohlenwasserstoffen durchgeführt.

Die Temperaturen können bei der Oxidation innerhalb eines bestimmten Bereiches variiert werden. Im allgemeinen arbeitet man zwischen — 20 °C und 130 °C, vorzugsweise zwischen 0 °C und 120 °C.

Bei der Durchführung der Oxidation setzt man die Ausgangsverbindung der Formel (Ic) im allgemeinen mit der jeweils berechneten Menge oder mit einem geringen Überschuß an Oxidationsmittel um. Die Aufarbeitung erfolgt jeweils nach üblichen Methoden.

Die bei der Durchführung des erfindungsgemäßen Verfahrens (b) als Ausgangsstoffe benötigten Phenol-Derivate sind durch die Formel (IV) allgemein definiert. In dieser Formel haben $R^3$, $R^4$ und p diejenigen Bedeutungen, die bereits im Zusammenhang mit der Beschreibung der Verbindungen der Formel (Ib) für diese Reste bzw. für diesen Index genannt wurden. $X^2$ steht vorzugsweise für Wasserstoff, Natrium oder Kalium.

Die Phenol-Derivate der Formel (IV) sind bekannt oder lassen sich nach bekannten Verfahren in einfacher Weise herstellen.

Die bei dem erfindungsgemäßen Verfahren (b) weiterhin als Ausgangsstoffe benötigten Halogenmethyl-phenylsulfide sind durch die Formel (V) allgemein definiert. In dieser Formel haben $R^2$, $R^5$ und m diejenigen Bedeutungen, die bereits im Zusammenhang mit der Beschreibung der Verbindungen der Formel (Ib) für diese Reste bzw. für diesen Index genannt wurden. Hal steht vorzugsweise für Chlor oder

Brom.

Die Halogenmethyl-phenylsulfide der Formel (V) sind bekannt oder lassen sich nach bekannten Verfahren in einfacher Weise herstellen (vgl. Liebigs Ann. *563*, 54 (1949)).

Als Säureakzeptoren können bei der Durchführung des erfindungsgemäßen Verfahrens (b) alle üblicherweise für derartige Umsetzungen geeigneten Säurebindemittel verwendet werden. Vorzugsweise in Frage kommen alle diejenigen Säureakzeptoren, die bereits im Zusammenhang mit der Beschreibung des erfindungsgemäßen Verfahrens (a) als bevorzugt verwendbare Säurebinder genannt wurden.

Als Verdünnungsmittel kommen bei der Durchführung des erfindungsgemäßen Verfahrens (b) praktisch alle inerten organischen Solventien in Betracht. Vorzugsweise verwendbar sind Alkohole, wie Methanol und Ethanol, weiterhin Ether, wie Dioxan und Tetrahydrofuran, außerdem Ketone, wie Aceton, ferner Amide, wie Dimethylformamid, und schließlich aliphatische oder aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol.

Die Reaktionstemperaturen können bei dem erfindungsgemäßen Verfahren (b) innerhalb eines größeren Bereiches variiert werden. Im allgemeinen arbeitet man bei Temperaturen zwischen 0 °C und 80 °C, vorzugsweise zwischen 20 °C und 60 °C.

Das erfindungsgemäße Verfahren (b) wird im allgemeinen bei Normaldruck durchgeführt. Es ist jedoch auch möglich, unter erhöhtem oder vermindertem Druck zur arbeiten.

Zur Durchführung des erfindungsgemäßen Verfahrens (b) werden die Ausgangsstoffe der Formeln (IV) und (V) im allgemeinen in etwa äquimolaren Mengen eingesetzt. Es ist jedoch auch möglich, eine der beiden Komponenten, vorzugsweise das Phenol-Derivat der Formel (IV), im Überschuß zu verwenden. Die Aufarbeitung erfolgt nach üblichen Methoden. Wurde bei der Umsetzung ein Überschuß an Phenol-Derivat der Formel (IV) angewandt, so empfiehlt es sich, überschüssiges Phenol-Derivat durch Schütteln des Reaktionsgemisches mit wäßriger Alkalimetallhydroxid-Lösung abzutrennen.

Ist bei dem erfindungsgemäßen Verfahren (b) die Herstellung von solchen Verbindungen der Formel (Ib) beabsichtigt, in denen n für 1 oder 2 steht, so führt man die Oxidation der Verbindungen der Formel (Ic) so durch, wie es bereits im Zusammenhang mit der Beschreibung des erfindungsgemäßen Verfahrens (a) erwähnt wurde.

Die bei dem erfindungsgemäßen Verfahren (c) als Ausgangsstoffe benötigten Sulfinsäuresalze sind durch die Formel (VI) allgemein definiert. In dieser Formel haben $R^2$, $R^5$ und m diejenigen Bedeutungen, die bereits im Zusammenhang mit der Beschreibung der Verbindungen der Formel (Ib) für diese Reste bzw. für diesen Index genannt wurden. M steht vorzugsweise für Natrium und Kalium.

Die Sulfinsäuresalze der Formel (VI) sind bekannt oder lassen sich nach bekannten Methoden in einfacher Weise herstellen (vgl. Houben-Weyl « Methoden der Organischen Chemie », Bd. IX, G. Thieme-Verlag, Stuttgart, 1955).

Die bei dem erfindungsgemäßen Verfahren (c) weiterhin als Ausgangsstoffe benötigten Halogenmethyl-phenyl-ether der Formel (III) wurden bereits im Zusammenhang mit der Beschreibung des erfindungsgemäßen Verfahrens (a) erwähnt.

Als Katalysatoren können bei dem erfindungsgemäßen Verfahren (c) alle für derartige Umsetzungen üblichen Reaktionsbeschleuniger verwendet werden. Vorzugsweise in Betracht kommen anorganische Jod-Verbindungen, wie Natriumiodid oder Kaliumiodid.

Als Verdünnungsmittel können bei der Durchführung des erfindungsgemäßen Verfahrens (c) alle inerten organischen Solventien verwendet werden. Vorzugsweise in Frage kommen Alkohole, wie Ethanol und Propanol, Ether, wie Dioxan und Tetrahydrofuran, ferner Kohlenwasserstoffe, wie Toluol, außerdem Halogenkohlenwasserstoffe, wie Chloroform, und außerdem Ketone, wie Aceton.

Die Reaktionstemperaturen können bei dem erfindungsgemäßen Verfahren (c) innerhalb eines größeren Bereiches variiert werden.

Im allgemeinen arbeitet man bei Temperaturen zwischen 20 °C und 100 °C, vorzugsweise zwischen 40 °C und 80 °C.

Das erfindungsgemäße Verfahren (c) wird im allgemeinen unter Normaldruck duchgeführt. Es ist jedoch auch möglich, unter erhöhtem oder vermindertem Druck zu arbeiten.

Zur Durchführung des erfindungsgemäßen Verfahrens (c) werden die Ausgangsstoffe der Formeln (VI) und (III) im allgemeinen in etwa äquimolaren Mengen eingesetzt. Es ist jedoch auch möglich, eine der beiden Komponenten, vorzugsweise das Sulfinsäuresalz der Formel (VI), im Überschuß zu werwenden. Bei Verwendung eines Katalysators setzt man pro Mol an Halogenmethyl-phenyl-ether der Formel (III) etwa 0,001 bis 0,1 Mol an Katalysator ein. Die Aufarbeitung und die Isolierung der Reaktionsprodukte erfolgen bei dem erfindungsgemäßen Verfahren (c) nach üblichen Methoden.

Die bei dem erfindungsgemäßen Verfahren (d) als Ausgangsstoffe benötigten Verbindungen sind durch die Formel (Ie) definiert. In dieser Formel steht n für 0, 1 oder 2. Diese Verbindungen sind bekannt oder lassen sich nach den oben angegebenen erfindungsgemäßen Verfahren herstellen.

Die bei dem erfindungsgemäßen Verfahren (d, Variante $\alpha$) als Reaktionskomponenten benötigten Verbindungen sind durch die Formel (VII) allgemein definiert. In dieser Formel steht $R^7$ für die oben angegebenen Reste.

Die Verbindungen der Formel (VII) sind bekannt.

Als Basen kommen bei der Durchführung des erfindungsgemäßen Verfahrens (d, Variante α) vorzugsweise Alkalihydroxide, wie Natriumhydroxid oder Kaliumhydroxid, und Alkalialkoholate, insbesondere Natrium- oder Kaliumalkoholate, in Betracht. Zweckmäßigerweise verwendet man in vielen Fällen das Kalium- oder Natrium-alkoholat desjenigen Alkohols der Formel (VII), der auch als Reaktionskomponente fungiert.

Als Verdünnungsmittel kommen bei der Durchführung des erfindungsgemäßen Verfahrens (d, Variante α) inerte organische Solventien, wie Methylenchlorid, Toluol, Xylol oder Ether, oder auch ein Überschuß der als Reaktionskomponente fungierenden Verbindung der Formel (VII) in Betracht.

Die Reaktionstemperaturen können bei dem erfindungsgemäßen Verfahren (d, Variante α) innerhalb eines größeren Bereiches variiert werden. Im allgemeinen arbeitet man bei Temperaturen zwischen 50 und 200 °C, vorzugsweise zwischen 80 und 160 °C.

Das erfindungsgemäße Verfahren (d, Variante α) wird entweder unter Normaldruck oder unter erhöhtem Druck durchgeführt. Zweckmäßigerweise arbeitet man in einem Druckgefäß unter dem jeweiligen Eigendruck des Reaktionsgemisches.

Bei der Durchführung des erfindungsgemäßen Verfahrens (d, Variante α) setzt man auf 1 Mol einer Verbindung der Formel (Ie) im allgemeinen einen großen Überschuß an einer Verbindung der Formel (VII) ein, so daß diese gleichzeitig als Verdünnungsmittel dienen kann. Außerdem gibt man eine katalytische Menge an Base hinzu.

In vielen Fällen empfiehlt es sich, die Base zu Beginn der Umsetzung dadurch herzustellen, daß man metallisches Natrium oder Kalium in der Verbindung der Formel (VII) auflöst und danach die Komponente der Formel (Ie) zugibt. Um einen möglichst vollständigen Umsatz zu erzielen, ist es von Vorteil, nach einigen Stunden das Verdünnungsmittel und die Verbindung der Formel (VII) abzuziehen und erneut durch eine entsprechende Menge der Komponente der Formel (VII) gegebenenfalls im Gemisch mit einem zusätzlichen Verdünnungsmittel zu ersetzen. Die Aufarbeitung erfolgt nach üblichen Methoden. Im allgemeinen geht man so vor, daß man das Reaktionsgemisch durch Abziehen der leicht flüchtigen Anteile unter vermindertem Druck einengt, den verbleibenden Rückstand mit Wasser und einem mit Wasser wenig mischbaren organischen Lösungsmittel versetzt, die organische Phase abtrennt, wäscht und nach gegebenenfalls vorheriger Filtration und Trocknung einengt.

Die bei den erfindungsgemäßen Verfahren (d, Variante β) als Reaktionskomponenten benötigten Basen sind durch die Formel (VIII) definiert.

Als Verdünnungsmittel kommen bei der Durchführung der ersten Stufe (Esterverseifung) des erfindungsgemäßen Verfahrens (d, Variante β) vorzugsweise aromatische Kohlenwasserstoffe, wie Toluol oder Xylol, ferner Alkohole, wie Methanol oder Ethanol, außerdem Ether, wie Dioxan, weiterhin Nitrile, wie Acetonitril, und auch Gemische aus organischen Solventien und Wasser in Betracht.

Die Temperaturen können bei der Durchführung der Esterverseifung in der ersten Stufe des erfindungsgemäßen Verfahrens (d, Variante β) innerhalb eines größeren Bereiches variiert werden. Im allgemeinen arbeitet man bei Temperaturen zwischen 0 °C und 160 °C, vorzugsweise zwischen 20 °C und 140 °C.

Bei der Esterverseifung in der ersten Stufe des erfindungsgemäßen Verfahrens (d, Variante β) können zur Acidifizierung alle üblichen starken Säuren eingesetzt werden. Vorzugsweise verwendbar sind Mineralsäuren, wie Salzsäure oder Schwefelsäure.

Bei der Durchführung der Esterverseifung in der ersten Stufe des erfindungsgemäßen Verfahrens (d, Variante β) geht man im allgemeinen so vor, daß man den Ester der Formel (Ie) in Gegenwart eines Verdünnungsmittels mit einer äquivalenten Menge oder mit einem Überschuß an Base bei der jeweils gewünschten Temperatur behandelt. Die Aufarbeitung erfolgt nach üblichen Methoden. Im allgemeinen geht man so vor, daß man das Reaktionsgemisch durch Abziehen des Lösungsmittels unter vermindertem druck einengt, den verbleibenden Rückstand in Wasser aufnimmt, mit Mineralsäure, wie zum Beispiel Salzsäure oder Schwefelsäure, ansäuert und die sich dabei abscheidende Säure abtrennt.

Wenn die Herstellung von Salzen beabsichtigt ist, so behandelt man die zunächst anfallenden Säuren in der zweiten Stufe des Verfahrens (d, Variante β) in Gegenwart eines Verdünnungsmittels mit der jeweils benötigten Base der Formel (VIII). Als Verdünnungsmittel kommen hierbei vorzugsweise Wasser oder mit Wasser mischbare organische Solventien in Frage.

Die Temperaturen können bei der Durchführung der zweiten Stufe des erfindungsgemäßen Verfahrens (d, Variante β) ebenfalls innerhalb eines größeren Bereiches variiert werden. Im allgemeinen arbeitet man bei Temperaturen zwischen 0 °C und 60 °C, vorzugsweise zwischen 10 °C und 50 °C.

Bei der Durchführung der zweiten Stufe des erfindungsgemäßen Verfahrens (d, Variante β) geht man im allgemeinen so vor, daß man die Säure der Formel (I) in wäßrigem Medium mit einer äquivalenten Menge an Base umsetzt. Zur Aufarbeitung wird das Reaktionsgemisch im allgemeinen eingedampft, und der verbleibende Rückstand wird getrocknet.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen

und von der Art der Applikation. In jedem Fall sollen Wachstumsregulatoren die Kulturpflanzen in bestimmter gewünschter Weise beeinflussen.

Pflanzenwuchsregulierende Stoffe können z. B. zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens (« Lagerns ») der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z. B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hydridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z. B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle, ist aber auch in anderen Kulturen wie z. B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden (« Ausdünnung »), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z. B. bei Tabak, Tomaten oder Kafee eine vollständige mechanische oder manuelle Beerntung in einem Arbeitsgang vorgenommen werden kann.

Durch Anwendung von Wachstumsregulatoren kann ferner die Samen- oder Knospenruhe der

Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemäß verwendbaren Wirkstoffe eignen sich nicht nur zur Regulierung des Pflanzenwachstums, sondern können darüber hinaus auch als Synergisten für Insektizide sowie zur Bekämpfung von Pilzen eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen in wesentlichen in Frage : Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid. Als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage ; z. B. nicht-ionogene und anionische Emulgatoren, wie Polyoxyethylen-Fettsäureester, Polyoxyethylen-Fettalkoholether, z. B. Alkylarylpolyglykol-ether, Alkylsulfonate, Alkylsulfate, Arylsulfonate sowie Eiweißhydrolysate.

Als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäß verwendbaren Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Staubemittel und Granulate, angewendet werden. Die Anwendung geschieht in üblicher Weise, z. B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Die Herstellung und die Verwendung der erfindungsgemäß verwendbaren Phenoxy-methan-Derivate gehen aus den nachfolgenden Beispielen hervor.

Herstellungsbeispiele

Beispiel 1

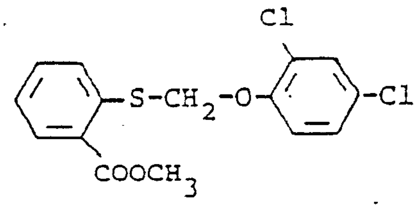

In eine Lösung von 33,6 g (0,2 Mol) 2-Mercapto-benzoesäure-methylester in 50 ml Tetrahydrofuran wurde bei 5 °C eine Lösung von 28 g (0,25 Mol) Kalium-tert.-butylat in 120 ml Tetrahydrofuran langsam eingetropft. Nach Beendigung des Eintropfens wurde noch 30 Minuten bei 5 °C weitergerührt. Das abgeschiedene Festprodukt wurde abgesaugt und in 100 ml Dimethylformamid gelöst. Zu dieser Lösung wurde unter Rühren bei 25 °C eine Lösung von 42,3 g (0,2 Mol) Chlormethyl-(2,4-dichlorphenyl)-ether in 60 ml Dimethylformamid zugetropft. Es wurde 1 Stunde bei 30 °C nachgerührt und dann aufgearbeitet, indem man das Reaktionsgemisch in 1,5 l Eiswasser einrührte. Die dabei entstehende Suspension wurde abgesaugt, und das abgeschiedene Festprodukt wurde an der Luft getrocknet. Man erhielt auf diese Weise 62 g (90,4 % der Theorie) an 1-(2,4-Dichlorphenoxy)-1-(2-methoxycarbonyl-phenylthio)-methan in Form farbloser Kristalle vom Schmelzpunkt 122-124 °C.

Beispiel 2

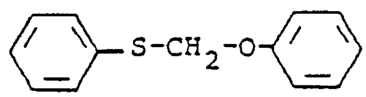

In eine Lösung von 40 g (0,34 Mol) Natriumphenolat in 200 ml Dimethylformamid wurde bei Raumtemperatur innerhalb von 30 Minuten unter Rühren eine Lösung von 47,5 g (0,3 Mol) Chlormethylphenylsulfid in 50 ml Dimethylformamid zugetropft. Dabei stieg die Temperatur des Reaktionsgemisches von 24 °C auf 35 °C an. Es wurde 14 Stunden bei Raumtemperatur nachgerührt und dann durch Abziehen des Lösungsmittels unter vermindertem Druck eingeengt. Das verbliebene Öl wurde nacheinander mit Wasser, 0,1 n wäßriger Natronlauge und Wasser gewaschen und nach dem Trocknen über Natriumsulfat einer fraktionierten Vakuumdestillation unterworfen. Man erhielt auf diese Weise 50 g (77,2 % der Theorie) an Phenoxy-phenylthiomethan in Form einer farblosen Flüssigkeit vom Siedepunkt 112-117 °C/0,16 mm Hg.

Beispiel 3

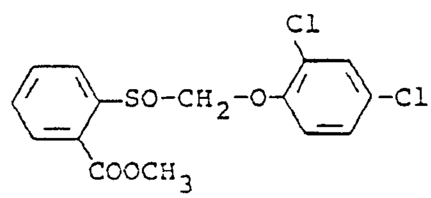

In eine Lösung von 17,1 g (0,05 Mol) 1-(2,4-Dichlorphenoxy)-1-(2-methoxycarbonyl-phenylthio)-methan in 100 ml Eisessig wurden bei 70 °C innerhalb von 30 Minuten unter Rühren 7 g (0,072 Mol) 35 %ige wäßrige Wasserstoffperoxid-Lösung eingetropft. Das Reaktionsgemisch wurde 1 Stunde bei 70 °C nachgerührt, dann auf Raumtemperatur abgekühlt und in 500 ml Eiswasser eingerührt. Die abgeschiedenen Kristalle wurden abgesaugt, mit Wasser gewaschen und im Exsikkator über Kaliumhydroxid getrocknet. Man erhielt auf diese Weise 17 g (95 % der Theorie) an 1-(2,4-Dichlor-phenoxy)-1-(2-methoxycarbonyl-phenylsulfinyl)-methan in Form farbloser Kristalle vom Schmelzpunkt 64-67 °C.

Beispiel 4

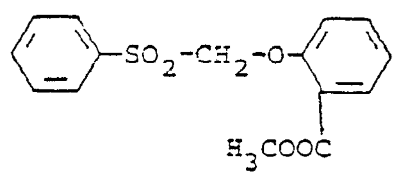

In eine Lösung von 48 g (0,175 Mol) 1-(2-Methoxycarbonylphenoxy)-1-phenylthio-methan in 180 ml Eisessig wurden bei 40-50 °C innerhalb von 30 Minuten unter Rühren 50 g (0,515 Mol) 35 %ige wäßrige Wasserstoffperoxid-Lösung eingetropft. Das Reaktionsgemisch wurde 14 Stunden bei 50 °C nachgerührt, dann abgekühlt und in 1 Liter Eiswasser eingerührt. Die abgeschiedenen Kristalle wurden abgesaugt und im Exsikkator über Kaliumhydroxid getrocknet. Man erhielt auf diese Weise 48,5 g (90,5 % der Theorie) an 1-(2-Methoxycarbonyl-phenoxy)-1-(phenylsulfonyl)-methan in Form farbloser Kristalle vom Schmelzpunkt 61-62 °C.

Beispiel 5

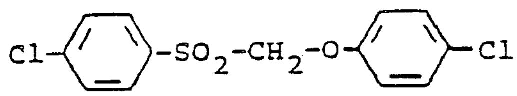

In eine Suspension von 4 g (0,021 Mol) Natrium-(4-chlorbenzol)-sulfinat in 50 ml Aceton wurden bei Raumtemperatur unter Rühren 2,84 g (0,02 Mol) Chlormethyl-(4-chlorphenyl)-ether zugetropft. Nach anschließender Zugabe einer katalytischen Menge Natriumiodid wurde zunächst 2 Stunden bei Raumtemperatur und dann 4 Stunden bei 50 °C nachgerührt. Zur Aufarbeitung wurde dann das Reaktionsgemisch durch Abziehen des Lösungsmittels unter vermindertem Druck eingeengt und der verbliebene ölige Rückstand in Methylenchlorid aufgenommen. Nach dem Waschen der organischen Phase mit Wasser wurde das Lösungsmittel unter vermindertem Druck abgezogen und der verbliebene Rückstand mit wenig Ethanol versetzt. In der Kälte schied sich ein Festprodukt aus, das abgesaugt und getrocknet wurde. Man erhielt auf diese Weise 0,84 g (15 % der Theorie) an 1-(4-Chlor-phenoxy)-1-(4-chlor-phenyl-sulfonyl)-methan in Form farbloser Kristalle vom Schmelzpunkt 114 °C.

Nach den in den Beispielen 1 bis 5 angegebenen Methoden wurden auch die in der folgenden Tabelle formelmäßig aufgeführten Phenoxy-methan-Derivate hergestellt.

Tabelle I

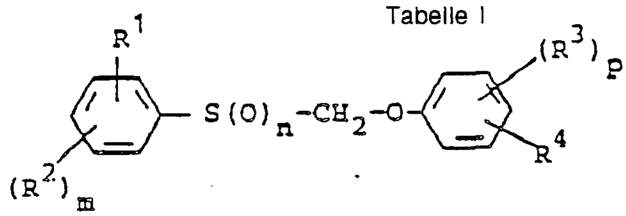

(I)

| Beispiel Nr. | $R^1$ | $R^2_m$ | $R^3_p$ | $R^4$ | n | Schmelzpunkt (°C) bzw. Siedepunkt bzw. Brechungsindex |
|---|---|---|---|---|---|---|
| 5 | 4-Cl | H | H | H | 0 | Kp 129/0,2 |
| 6 | 4-$CH_3$ | H | H | H | 0 | Kp 110-112/0,16 |
| 7 | 4-Cl | H | 2-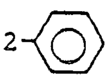 | H | 0 | Fp 38-40 °C |
| 8 | 4-Cl | H | H | H | 2 | Fp 78-79 °C |
| 9 | 4-$CH_3$ | H | H | H | 2 | Fp 73-75 °C |
| 10 | H | H | H | H | 2 | Fp 58-60 °C |
| 11 | 4-Cl | H | 2-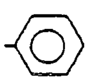 | H | 2 | Fp 113-114 °C |
| 12 | 4-Cl | H | 4-Cl | 3-$CH_3$ | 0 | $n_D^{20}$ 1,6160 |
| 13 | 4-$CH_3$ | H | 4-Cl | 3-$CH_3$ | 0 | $n_D^{20}$ 1,6300 |
| 14 | H | H | 4-Cl | 3-$CH_3$ | 0 | $n_D^{20}$ 1,6300 |
| 15 | 4-Cl | H | 4-Cl | H | 0 | $n_D^{20}$ 1,6211 |
| 16 | 4-$CH_3$ | H | 4-Cl | H | 0 | Fp 41-43 °C |
| 17 | H | H | 4-Cl | H | 0 | $n_D^{20}$ 1,6125 |

Tabelle I (Fortsetzung)

| Beispiel Nr. | $R^1$ | $R^2_m$ | $R^3_p$ | $R^4$ | n | Schmelzpunkt (°C) bzw. Siedepunkt bzw. Brechungsindex |
|---|---|---|---|---|---|---|
| 18 | 4-Cl | H | 4-Cl | 3-$CH_3$ | 2 | Fp 100-101°C |
| 19 | 4-$CH_3$ | H | 4-Cl | 3-$CH_3$ | 2 | Fp 99-100°C |
| 20 | H | H | 4-Cl | 3-$CH_3$ | 2 | Fp 99-100°C |
| 21 | 4-$CH_3$ | H | 4-Cl | H | 2 | Fp 88°C |
| 22 | H | H | 4-Cl | H | 2 | Fp 99°C |
| 23 | H | H | 2- 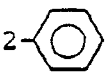 | H | 0 | $n_D^{20}$ 1,6435 |
| 24 | 4-$CH_3$ | H | 2- 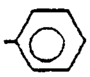 | H | 0 | $n_D^{20}$ 1,6342 |
| 25 | H | H | 2- 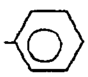 | H | 2 | Fp 102-103°C |
| 26 | 4-$CH_3$ | H | 2- 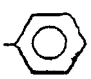 | H | 2 | Fp 120-121°C |
| 27 | 4-$C_4H_9$- tert. | H | H | H | 0 | $n_D^{20}$ 1,5905 |
| 28 | " | H | 4-Cl | 3-$CH_3$ | 0 | $n_D^{20}$ 1,6030 |
| 29 | " | H | H | H | 2 | Fp 63-65°C |
| 30 | " | H | 4-Cl | 3-$CH_3$ | 2 | Fp 80-84°C |
| 31 | 4-Cl | H | 2-$OC_3H_7$- iso | H | 0 | $n_D^{20}$ 1,5880 |
| 32 | 2-$COOCH_3$ | H | 4-Cl | H | 0 | Fp 59°C |
| 33 | 4-$CH_3$ | H | 3-$CH_3$ | H | 0 | Kp 127-128/0,5 |
| 34 | 4-$CH_3$ | H | 2-Cl | 4-Cl | 0 | Fp 40-41°C |
| 35 | 4-$CH_3$ | H | 2-$OC_3H_7$- iso | H | 0 | $n_D^{20}$ 1,5805 |
| 36 | 4-Cl | H | H | 2-COO $CH_3$ | 2 | Fp 90-91°C |
| 37 | 4-Cl | H | 2-$OC_3H_7$- iso | H | 2 | Fp 72°C |
| 38 | 4-$CH_3$ | H | 3-$CH_3$ | H | 2 | Fp 52°C |
| 39 | 4-$CH_3$ | H | 2-Cl | 4-Cl | 2 | Fp 91°C |
| 40 | 4-$CH_3$ | H | H | 2-COO $CH_3$ | 0 | Fp 63-65°C |
| 41 | H | H | H | 2-COO $CH_3$ | 0 | Fp 41-43°C |
| 42 | 4-Cl | H | 3-$OCH_3$ | H | o | $n_D^{20}$ 1,6230 |
| 43 | 4-$CH_3$ | H | H | 2-COO $CH_3$ | 2 | Fp 126-128°C |

Tabelle I (Fortsetzung)

| Beispiel Nr. | $R^1$ | $R^2_m$ | $R^3_p$ | $R^4$ | n | Schmelzpunkt (°C) bzw. Siedepunkt bzw. Brechungsindex |
|---|---|---|---|---|---|---|
| 44 | 2-COOCH$_3$ | H | 2-Cl | 4-Cl | 2 | Fp 109-110°C |
| 45 | 3-Cl | 4-Cl | H | 2-COOCH$_3$ | 0 | $n_D^{20}$ 1,6109 |
| 46 | 2-Cl | 5-Cl | H | 2-COOCH$_3$ | 0 | Fp 46-48°C |
| 47 | 4-$C_4H_9$-tert. | H | H | 2-COOCH$_3$ | 0 | $n_D^{20}$ 1,5722 |
| 48 | H | H | H | 2-COOC$_2H_5$ | 0 | $n_D^{20}$ 1,5881 |
| 49 | H | H | H | 2-COO-$C_4H_9$-iso | 0 | $n_D^{20}$ 1,5720 |
| 50 | H | H | H | 2-COO-$C_3H_7$-iso | 0 | $n_D^{20}$ 1,5721 |
| 51 | H | H | H | 2-COO-$C_5H_{11}$-iso | 0 | $n_D^{20}$ 1,5650 |
| 52 | H | H | H | 2-COO-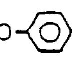 | 0 | $n_D^{20}$ 1,6052 |
| 53 | H | H | H | 2-CO-NH$_2$ | 0 | Fp 127-128°C |
| 54 | H | H | H | 4-COOCH$_3$ | 0 | Fp 67-68°C |
| 55 | H | H | H | 4-COO-$C_2H_5$ | 0 | $n_D^{20}$ 1,5716 |
| 56 | H | H | H | 4-COO-$C_3H_7$-n | 0 | $n_D^{20}$ 1,5690 |
| 57 | H | H | 4-NO$_2$ | 2-CN | 0 | Fp 72-74°C |
| 58 | H | H | 2,6-Br$_2$ | 4-CN | 0 | Fp 85-87°C |
| 59 | H | H | H | 4-CN | 0 | $n_D^{20}$ 1,5521 |
| 60 | H | H | H | 2-COOCH$_3$ | 1 | $n_D^{20}$ 1,5800 |
| 61 | H | H | H | 2-COOC$_2H_5$ | 1 | $n_D^{20}$ 1,5760 |
| 62 | H | H | H | 2-COOC$_2H_5$ | 2 | $n_D^{20}$ 1,5597 |

Tabelle I (Fortsetzung)

| Beispiel Nr. | $R^1$ | $R^2_m$ | $R^3_p$ | $R^4$ | n | Schmelzpunkt (°C) bzw. Siedepunkt bzw. Brechungsindex |
|---|---|---|---|---|---|---|
| 63 | H | H | H | $2-COOC_3H_7$-iso | 1 | $n_D^{20}$ 1,5624 |
| 64 | H | H | H | $2-COOC_3H_7$-iso | 2 | Fp 55 - 57°C |
| 65 | H | H | H | $2-COOC_5H_{11}$-iso | 1 | $n_D^{20}$ 1,5600 |
| 66 | H | H | H | $2-COOC_5H_{11}$-iso | 2 | $n_D^{20}$ 1,5413 |
| 67 | H | H | $6-CH_3$ | $2-COOCH_3$ | 0 | $n_D^{20}$ 1,5890 |
| 68 | H | H | $6-CH_3$ | $2-COOCH_3$ | 1 | $n_D^{20}$ 1,5822 |

Beispiel 69

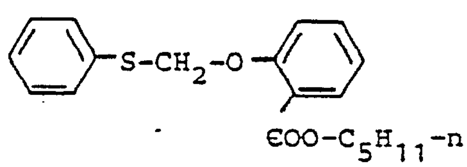

0,5 g (0,022 Mol) Natrium wurden in 250 ml wasserfreiem n-Amylalkohol gelöst. Danach wurde diese Lösung zusammen mit 25 g (0,091 Mol) 2-(Phenylthio-methoxy)-benzoesäuremethylester in einem 0,7 l fassenden Autoklaven aus VA-Stahl gegeben und 18 Stunden auf 140 °C erhitzt. Anschließend wurde das Reaktionsgemisch entnommen und unter vermindertem Druck eingeengt. Man versetzte erneut mit 250 ml wasserfreiem n-Amylalkohol und erhitzte wiederum 18 Stunden im Autoklaven auf 140 °C. Nach nochmaliger Wiederholung dieses Vorganges wurde aufgearbeitet, indem man das Reaktionsgemisch unter vermindertem Druck eingedampfte und den Rückstand mit Wasser und Methylenchlorid aufnahm. Die organische Phase wurde abgetrennt, mit Wasser neutral gewaschen, über eine dünne Schicht Kieselgel 60, Korngröße 0,063-0,2 mm, filtriert und durch Abziehen des Lösungsmittels unter vermindertem Druck eingeengt. Man erhielt auf diese Weise 24,3 g (80,8 % der Theorie) an 2-(Phenylthio-methoxy)-benzoesäure-n-amylester in Form einer Flüssigkeit mit dem Brechungsindex $n_D^{20}$ = 1,557 5.

Beispiel 70

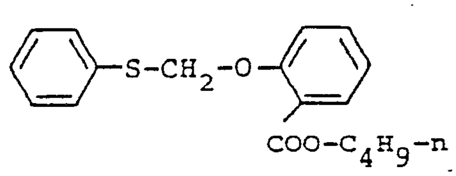

0,5 g (0,022 Mol) Natrium wurden in 250 ml wasserfreiem n-Butanol gelöst. Danach wurde diese Lösung zusammen mit 25 g (0,091 Mol) 2-(Phenylthio-methoxy)-benzoesäuremethylester in einen 0,7 l fassenden Autoklaven aus VA-Stahl gegeben und 2 Stunden auf 120 °C erhitzt. Danach wurde aufgearbeitet, indem man das Reaktionsgemisch unter vermindertem Druck eindampfte und den Rückstand mit Wasser und Methylenchlorid aufnahm. Die organische Phase wurde abgetrennt, mit Wasser neutral gewaschen und über eine dünne Schicht Kieselgel 60, Korngröße 0,063-0,2 mm, filtriert

und durch Abziehen des Lösungsmittels unter vermindertem Druck eingeengt. Man erhielt auf diese Weise 22 g (76,5 % der Theorie) an 2-(Phenylthio-methoxy)-benzoesäure-n-butylester in Form einer Flüssigkeit mit dem Brechungsindex $n_D^{20}$ = 1,577 5.

Nach der in den Beispielen 69 und 70 angegebenen Methode wurden auch die in der folgenden Tabelle formelmäßig aufgeführten Phenoxy-methan-Derivate hergestellt :

Tabelle 2

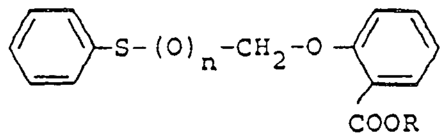

(Ia)

| Beispiel Nr. | R | n | Brechungsindex bzw. Schmelzpunkt |
|---|---|---|---|
| 71 | $-C_3H_7-n$ | 0 | $n_D^{20}$ = 1,5828 |
| 72 | $-C_8H_{17}-n$ | 0 | $n_D^{20}$ = 1,5348 |
| 73 | $-C_6H_{13}-n$ | 0 | $n_D^{20}$ = 1,5430 |
| 74 | $-CH_2-CH_2-O-CH_3$ | 0 | Fp. = 40 - 42°C |
| 75 | $-CH_2-CH_2-O-C_4H_9-n$ | 0 | $n_D^{20}$ = 1,5620 |
| 76 | $-CH_2-CH_2-O-C_2H_5$ | 0 | $n_D^{20}$ = 1,5682 |
| 77 | $-CH_2-CH=CH_2$ | 0 | $n_D^{20}$ = 1,5952 |
| 78 | $-CH_2-CH_2-S-C_2H_5$ | 0 | $n_D^{20}$ = 1,5390 |
| 79 | $-(CH_2)_2-O-(CH_2)_2-OCH_3$ | 0 | $n_D^{20}$ = 1,5621 |
| 80 | $-(CH_2)_2-O-C_3H_7-iso$ | 0 | $n_D^{20}$ = 1,5520 |

Beispiel 81

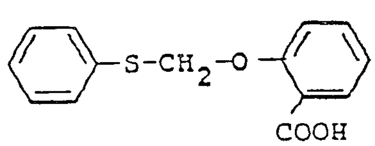

Zu einer Lösung von 33,6 g (0,6 Mol) Kaliumhydroxid in 500 ml Methanol wurden 137 g (0,5 Mol) 2-(Phenylthio-methoxy)-benzoesäure-methylester gegeben, und es wurde 3 Stunden unter Rühren zum Sieden erhitzt. Danach wurde aufgearbeitet, indem man das Lösungsmittel unter vermindertem Druck abzog, den Rückstand in 1 l Wasser aufnahm, und die entstehende Lösung kurz mit Ether extrahierte.

Anschließend wurde die wäßrige Phase mit 300 ml Ether überschichtet und unter Umschütteln durch Zugabe von 2 normaler wäßriger Schwefelsäure auf einem pH-Wert von 2 angesäuert. Die Phasen wurden getrennt, und die wäßrige Phase wurde noch zweimal mit je 300 ml Ether extrahiert. Nach dem Trocknen der vereinigten organischen Phasen über Natriumsulfat wurde durch Abziehen des Lösungsmittels unter vermindertem Druck eingeengt. Man erhielt auf diese Weise 126 g (96,9 % der Theorie) an 2-(Phenylthio-methoxy)-benzoesäure in Form eines farblosen Öles, das beim Anreiben durchkristallisierte. Fp. = 57-59 °C.

Beispiel 82

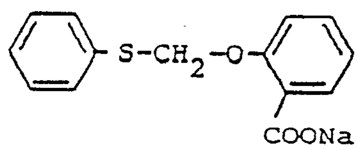

126 g (0,48 Mol) 2-(Phenylthio-methoxy)-benzoesäure wurden unter Zusatz von 19,18 g (0,48 Mol) Natriumhydroxid in 500 ml Wasser gelöst. Das Reaktionsgemisch wurde 30 Minuten bei Raumtemperatur gerührt und dann bei 60 °C unter vermindertem Druck vollständig eingedampft. Das zurückbleibende farblose, pulverförmige Produkt wurde 16 Stunden bei 100 °C unter vermindertem Druck getrocknet. Man erhielt auf diese Weise 133,5 g (98,6 % der Theorie) an 2-(Phenylthio-methoxy)-benzoesäure-Natriumsalz vom Schmelzpunkt 204-207 °C.

Beispiel 83

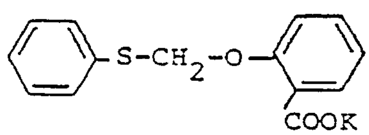

Nach der im Beispiel 82 angegebenen Methode wurde auch das 2-(Phenylthio-methoxy)-benzoesäure-Kaliumsalz hergestellt. Schmelzpunkt : 240 °C (Zers.).

Nach den in den Beispielen 3 und 4 angegebenen Methoden wurden weiterhin die in Tabelle 3 formelmäßig aufgeführten Phenoxy-methan-Derivate hergestellt.

Tabelle 3

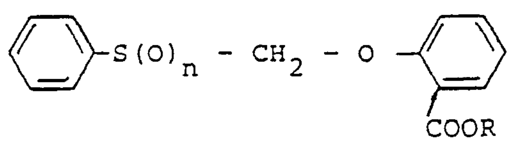

(Ia)

| Beispiel Nr. | R | n | Brechungsindex bzw. [$n_D^{20}$] Schmelzpunkt [°C] |
|---|---|---|---|
| 84 | $-CH_2-CH=CH_2$ | 1 | 1,5926 |
| 85 | $-CH_2-CH=CH_2$ | 2 | 1,5703 |
| 86 | $-(CH_2)_7-CH_3$ | 2 | 1,5220 |
| 87 | $-(CH_2)_7-CH_3$ | 1 | 1,5430 |
| 88 | $-(CH_2)_5-CH_3$ | 1 | 1,5290 |
| 89 | $-(CH_2)_5-CH_3$ | 2 | 52 - 54 |
| 90 | $-CH_2CH_2-OCH_3$ | 2 | 1,5625 |
| 91 | $-CH_2CH_2-OCH_3$ | 1 | 1,5765 |
| 92 | $-CH_2CH_2OCH_2CH_2-OCH_3$ | 1 | 1,5668 |
| 93 | $-CH_2CH_2OCH_2CH_2-OCH_3$ | 2 | 1,5490 |
| 94 | $-CH_2CH_2-OCH(CH_3)_2$ | 1 | 1,5530 |
| 95 | $-CH_2CH_2-OCH(CH_3)_2$ | 2 | 1,5350 |

Tabelle 3 (Fortsetzung)

| Beispiel Nr. | R | n | Brechungsindex bzw. $[n_D^{20}]$ Schmelzpunkt [°C] |
|---|---|---|---|
| 96 | H | 1 | 107 - 8 |
| 97 | H | 2 | 139 - 40 |
| 98 | K | 2 | 184 - 5 |
| 99 | Na | 2 | 105 - 7 |
| 100 | Na | 1 | 97 - 99 |
| 101 | K | 1 | 125 - 7 |

Beispiel A

Stimulierung der $CO_2$-Fixierung bei Sojabohnen

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Im weiteren Verlauf des Experimentes wird die $CO_2$-Fixierung der Pflanzen mit üblichen Methoden gemessen. Die Werte werden mit denen nicht mit Wirkstoffen behandelter Kontrollpflanzen verglichen.

Es bedeuten :

— : Hemmung der $CO_2$-Fixierung
0 : $CO_2$-Fixierung wie bei der Kontrolle
+ : geringe Stimulierung der $CO_2$-Fixierung
++ : starke Stimulierung der $CO_2$-Fixierung
+++ : sehr starke Stimulierung der $CO_2$-Fixierung

Wirkstoffe, Wirkstoffkonzentrationen und Versuchsergebnisse gehen aus der folgenden Tabelle hervor.

Tabelle A

Stimulierung der $CO_2$-Fixierung bei Sojabohnen

| Wirkstoff | Wirkstoffkonzentration in % | Wirkung |
|---|---|---|
| (13) | 0,05 | + |
| (15) | 0,05 | + |
| (18) | 0,05 | + |
| (41) | 0,05 | + |
| (44) | 0,05 | + |
| (46) | 0,05 | ++ |
| (74) | 0,05 | + |
| (77) | 0,05 | ++ |

Tabelle A (Fortsetzung)

| Wirkstoff | Wirkstoffkonzentration in % | Wirkung |
|---|---|---|
| (80) | 0,05 | ++ |
| – (Kontrolle) | – | 0 |

Beispiel B

Wuchshemmung bei Gerste

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Wirkstoffe, Wirkstoffkonzentrationen und Versuchsergebnisse gehen aus der folgenden Tabelle hervor.

Tabelle B

Wuchshemmung bei Gerste

| Wirkstoff | Wirkstoffkonzentration in % | Wuchshemmung in % |
|---|---|---|
| (4) | 0,05 | 30 |
| (41) | 0,05 | 45 |
| (48) | 0,05 | 60 |
| (50) | 0,05 | 51 |
| (51) | 0,05 | 42 |
| (52) | 0,05 | 42 |
| (60) | 0,05 | 31 |
| (74) | 0,05 | 71 |
| (75) | 0,05 | 49 |
| (76) | 0,05 | 51 |
| (77) | 0,05 | 54 |
| (78) | 0,05 | 21 |
| (79) | 0,05 | 37 |
| (80) | 0,05 | 37 |
| (81) | 0,05 | 23 |
| (82) | 0,05 | 68 |

Tabelle B (Fortsetzung)

| Wirkstoff | Wirkstoffkonzentration in % | Wuchshemmung in % |
|---|---|---|
| (83) | 0,05 | 19 |
| - (Kontrolle | - | 0 |

Beispiel C

Wuchshemmung bei Baumwolle

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Wirkstoffe, Wirkstoffkonzentrationen und Versuchsergebnisse gehen aus der folgenden Tabelle hervor.

Tabelle C

Wuchshemmung bei Baumwolle

| Wirkstoff | Wirkstoffkonzentration in % | Wuchshemmung in % |
|---|---|---|
| (30) | 0,05 | 33 |
| (59) | 0,05 | 72 |
| - (Kontrolle) | - | 0 |

Beispiel D

Ethylenbildung

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Aus Sojabohnenblättern werden Blattstücke gleicher Größe gestanzt. Diese werden zusammen mit 1 ml Wirkstoffzubereitung bzw. Kontrollösung in luftdicht verschließbare Gefäße gegeben. Nach 24 Stunden wird das Ethylen, das sich in den Gefäßen gesammelt hat, mit üblichen Nachweismethoden bestimmt. Die Ethylenentwicklung der mit den Wirkstoffzubereitungen behandelten Blattstücke wird mit derjenigen der Kontrollen verglichen.

Es bedeuten :

0 : Ethylenentwicklung wie bei der Kontrolle
+ : leicht erhöhte Ethylenentwicklung
++ : stark erhöhte Ethylenentwicklung
+++ : sehr stark erhöhte Ethylenentwicklung

Wirkstoffe, Wirkstoffkonzentrationen und Versuchsergebnisse gehen aus der folgenden Tabelle hervor.

Tabelle D

Ethylenbildung

| Wirkstoff | Wirkstoffkonzentration in % | Wirkung |
|---|---|---|
| (41) | 0,001 | +++ |
| (50) | 0,001 | ++ |
| (52) | 0,001 | + |
| (56) | 0,001 | + |
| – (Kontrolle) | – | 0 |

Beispiel E

Wuchsbeeinflussung bei Zuckerrüben

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitungen besprüht. Nach 14 Tagen wird der Zuwachs der Pflanzen gemessen und die Wuchsbeeinflussung in Prozent des Zuwachses der Kontrollpflanzen berechnet. Es bedeuten 0 % Wuchsbeeinflussung ein Wachstum entsprechend dem der Kontrollpflanzen. Negative Werte kennzeichnen eine Wuchshemmung, positive Werte eine Wuchsförderung gegenüber den Kontrollpflanzen.

Wirkstoffe, Wirkstoffkonzentrationen und Versuchsergebnisse gehen aus der folgenden Tabelle hervor.

Tabelle E

Wuchsbeeinflussung bei Zuckerrüben

| Wirkstoff | Wirkstoffkonzentration in % | Wuchsbeeinflussung in % |
|---|---|---|
| (5) | 0,05 | + 15 |
| (52) | 0,05 | + 13 |
| (78) | 0,05 | + 12 |
| | 0,003 | + 12 |
| (79) | 0,05 | + 4 |
| | 0,003 | + 12 |
| (80) | 0,05 | + 12 |
| – (Kontrolle) | – | 0 |

**Patentansprüche**

1. Verwendung von Phenoxymethan-Derivaten der Formel

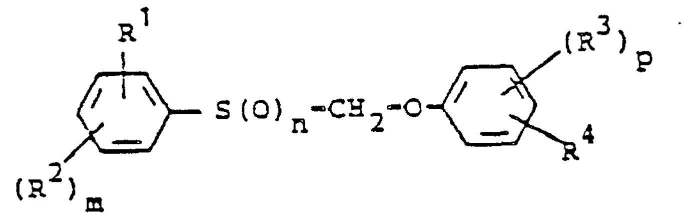

(I)

in welcher

$R^1$ für Wasserstoff, geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 5 Halogenatomen, geradkettiges oder verzweigtes Alkoxy mit 1 bis 6 Kohlenstoffatomen, Halogenalkoxy mit 1 bis 6 Kohlenstoffatomen und 1 bis 5 Halogenatomen, Alkoxycarbonyl mit 1 bis 10 Kohlenstoffatomen in der Alkoxygruppe, Cycloalkyloxycarbonyl mit 3 bis 8 Kohlenstoffatomen in der Cycloalkylgruppe, Fluor, Chlor, Brom, Jod oder Nitro steht,

$R^2$ für Wasserstoff, geradkettiges oder verzweigtes Alkyl mit 1 bis 4 Kohlenstoffatomen, Fluor, Chlor, Brom oder Jod steht,

$R^3$ für Wasserstoff, geradkettiges oder verzweigtes Alkyl mit 1 bis 6 Kohlenstoffatomen, geradkettiges oder verzweigtes Alkenyl mit 3 bis 6 Kohlenstoffatomen, geradkettiges oder verzweigtes Alkinyl mit 3 bis 6 Kohlenstoffatomen, gegebenenfalls durch Alkyl mit 1 bis 4 Kohlenstoffatomen, Fluor, Chlor, Brom und/oder Jod substituiertes Phenyl, gegebenenfalls durch Alkyl mit 1 bis 4 Kohlenstoffatomen, Fluor, Chlor, Brom und/oder Jod substituiertes Benzyl, Alkoxy mit 1 bis 6 Kohlenstoffatomen, Phenoxy, Halogenalkyl mit 1 bis 6 Kohlenstoffatomen und 1 bis 5 Halogenatomen, Halogenalkoxy mit 1 bis 6 Kohlenstoffatomen und 1 bis 5 Halogenatomen, Alkylthio mit 1 bis 6 Kohlenstoffatomen, Halogenalkylthio mit 1 bis 6 Kohlenstoffatomen und 1 bis 5 Halogenatomen, Alkylsulfonyl mit 1 bis 6 Kohlenstoffatomen, Phenylthio, Phenylsulfonyl, Nitro, FLuor, Chlor, Brom oder Jod steht,

$R^4$ für Wasserstoff, geradkettiges oder verzweigtes Alkyl mit 1 bis 6 Kohlenstoffatomen, Fluor, Chlor, Brom, Jod, Cycloalkyloxycarbonyl mit bis zu 10 Kohlenstoffatomen in der Cycloalkylgruppe, Aminocarbonyl, Alkylaminocarbonyl mit 1 bis 6 Kohlenstoffatomen in der Alkylgruppe, Dialkylaminocarbonyl mit 1 bis 6 Kohlenstoffatomen in jeder Alkylgruppe, Cycloalkylaminocarbonyl mit 3 bis 8 Kohlenstoffatomen in der Cycloalkylgruppe, Dicycloalkyl-aminocarbonyl mit 3 bis 8 Kohlenstoffatomen in jeder Cycloalkylgruppe, gegebenenfalls durch Alkyl mit 1 bis 4 Kohlenstoffatomen, Fluor, Chlor, Brom und/oder Jod substituiertes Phenoxycarbonyl, für Cyano oder den Rest der Formel $-COOR$ steht, worin

R für Wasserstoff, ein Natrium- oder Kalium-Kation, ein Äquivalent eines Magnesium- oder Calcium-Kations, für Alkyl mit 1 bis 10 Kohlenstoffatomen, Alkenyl mit 2 bis 10 Kohlenstoffatomen, Alkoxyalkyl mit 1 bis 6 Kohlenstoffatomen in der Alkoxygruppe und 1 bis 6 Kohlenstoffatomen im Alkylteil, Alkylthioalkyl mit 1 bis 6 Kohlenstoffatomen in der Alkylthiogruppe und 1 bis 6 Kohlenstoffatomen im Alkylteil oder für den Rest der Formel $-CH_2-O-(CH_2-CH_2-O)_q$-Alk steht, worin

Alk für Alkyl mit 1 bis 6 Kohlenstoffatomen steht und

q für 0, 1 oder 2 steht,

m für 1 oder 2 steht,

n für 0, 1 oder 2 steht und

p für 1 oder 2 steht,

zur Regulierung des Pflanzenwachstums.

2. Phenoxymethan-Derivate der Formel

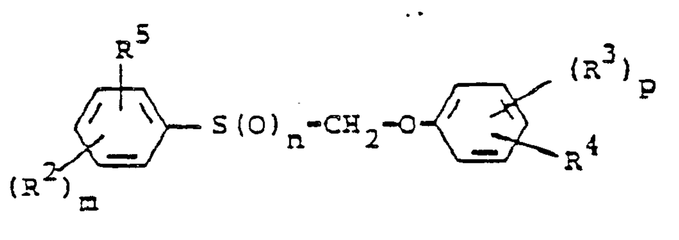

(Ib)

in welcher

$R^5$ für geradkettiges oder verzweigtes Alkyl mit 2 bis 4 Kohlenstoffatomen, geradkettiges oder verzweigtes Alkoxy mit 1 bis 4 Kohlenstoffatomen, Halogenalkyl mit 1 bis 3 Kohlenstoffatomen und 1 bis 5 Fluor-, Chlor- und/oder Brom-Atomen, Halogenalkoxy mit 1 bis 3 Kohlenstoffatomen und 1 bis 5 Fluor-, Chlor- und/oder Brom-Atomen, Alkoxycarbonyl mit 1 bis 4 Kohlenstoffatomen in der Alkoxygruppe,

Cycloalkyloxycarbonyl mit 3 bis 6 Kohlenstoffatomen in der Cycloalkylgruppe, Fluor, Brom oder Nitro steht,

$R^2$ für Wasserstoff, geradkettiges oder verzweigtes Alkyl mit 1 bis 4 Kohlenstoffatomen, Fluor, Chlor oder Brom steht,

$R^3$ für Wasserstoff, geradkettiges oder verzweigtes Alkyl mit 1 bis 4 Kohlenstoffatomen, geradkettiges oder verzweigtes Alkenyl mit 3 bis 5 Kohlenstoffatomen, geradkettiges oder verzweigtes Alkinyl mit 3 bis 5 Kohlenstoffatomen, gegebenenfalls durch Alkyl mit 1 bis 3 Kohlenstoffatomen, Fluor, Chlor und/oder Brom substituiertes Phenyl, gegebenenfalls durch Alkyl mit 1 bis 3 Kohlenstoffatomen, Fluor, Chlor und/oder Brom substituiertes Benzyl, Alkoxy mit 1 bis 4 Kohlenstoffatomen, Phenoxy, Halogenalkyl mit 1 bis 3 Kohlenstoffatomen und 1 bis 5 Fluor-, Chlor- und/oder Brom-Atomen, Halogenalkoxy mit 1 bis 3 Kohlenstoffatomen und 1 bis 5 Fluor-, Chlor- und/oder Brom-Atomen, Alkylthio mit 1 bis 4 Kohlenstoffatomen, Halogenalkylthio mit 1 bis 3 Kohlenstoffatomen und 1 bis 5 Fluor-, Chlor- und/oder Brom-Atomen, Alkylsulfonyl mit 1 bis 4 Kohlenstoffatomen, Phenylthio, Phenylsulfonyl, Nitro, Fluor, Chlor oder Brom steht,

$R^4$ für Wasserstoff, geradkettiges oder verzweigtes Alkyl mit 1 bis 4 Kohlenstoffatomen, Fluor, Chlor, Brom, Alkoxycarbonyl mit 1 bis 8 Kohlenstoffatomen in der Alkoxygruppe, Cycloalkyloxycarbonyl mit 3 bis 8 Kohlenstoffatomen in der Cycloalkylgruppe, Aminocarbonyl, Alkylaminocarbonyl mit 1 bis 4 Kohlenstoffatomen in der Alkylgruppe, Dialkylaminocarbonyl mit 1 bis 4 Kohlenstoffatomen in jeder Alkylgruppe, Cycloalkylaminocarbonyl mit 3 bis 7 Kohlenstoffatomen in der Cycloalkylgruppe, Dicycloalkyl-aminocarbonyl mit 3 bis 7 Kohlenstoffatomen in jeder Cycloalkylgruppe, gegebenenfalls durch Alkyl mit 1 bis 3 Kohlenstoffatomen, Fluor, Chlor und/oder Brom substituiertes Phenoxycarbonyl oder für Cyano steht,

m für 1 oder 2 steht,

n für 1 oder 2 steht und

p für 1 oder 2 steht, und

$R^5$ außerdem auch für Wasserstoff oder Chlor steht, sofern $R^3$ nicht für Wasserstoff, Chlor, Methyl oder Nitro steht.

3. Verfahren zur Herstellung von Phenoxymethan-Derivaten der Formel

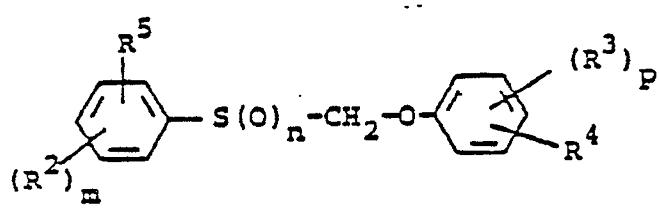

(Ib)

in welcher

$R^5$ für geradkettiges oder verzweigtes Alkyl mit 2 bis 4 Kohlenstoffatomen, geradkettiges oder verzweigtes Alkoxy mit 1 bis 4 Kohlenstoffatomen, Halogenalkyl mit 1 bis 3 Kohlenstoffatomen und 1 bis 5 Fluor-, Chlor- und/oder Brom-Atomen, Halogenalkoxy mit 1 bis 3 Kohlenstoffatomen und 1 bis 5 Fluor-, Chlor- und/oder Brom-Atomen, Alkoxycarbonyl mit 1 bis 4 Kohlenstoffatomen in der Alkoxygruppe, Cycloalkyloxycarbonyl mit 3 bis 6 Kohlenstoffatomen in der Cycloalkylgruppe, Fluor, Brom oder Nitro steht,

$R^2$ für Wasserstoff, geradkettiges oder verzweigtes Alkyl mit 1 bis 4 Kohlenstoffatomen, Fluor, Chlor oder Brom steht,

$R^3$ für Wasserstoff, geradkettiges oder verzweigtes Alkyl mit 1 bis 4 Kohlenstoffatomen, geradkettiges oder verzweigtes Alkenyl mit 3 bis 5 Kohlenstoffatomen, geradkettiges oder verzweigtes Alkinyl mit 3 bis 5 Kohlenstoffatomen, gegebenenfalls durch Alkyl mit 1 bis 3 Kohlenstoffatomen, Fluor, Chlor und/oder Brom substituiertes Phenyl, gegebenenfalls durch Alkyl mit 1 bis 3 Kohlenstoffatomen, Fluor, Chlor und/oder Brom substituiertes Benzyl, Alkoxy mit 1 bis 4 Kohlenstoffatomen, Phenoxy, Halogenalkyl mit 1 bis 3 Kohlenstoffatomen und 1 bis 5 Fluor-, Chlor- und/oder Brom-Atomen, Halogenalkoxy mit 1 bis 3 Kohlenstoffatomen und 1 bis 5 Fluor-, Chlor- und/oder Brom-Atomen, Alkylthio mit 1 bis 4 Kohlenstoffatomen, Halogenalkylthio mit 1 bis 3 Kohlenstoffatomen und 1 bis 5 Fluor-, Chlor- und/oder Brom-Atomen, Alkylsulfonyl mit 1 bis 4 Kohlenstoffatomen, Phenylthio, Phenylsulfonyl, Nitro, Fluor, Chlor oder Brom steht,

$R^4$ für Wasserstoff, geradkettiges oder verzweigtes Alkyl mit 1 bis 4 Kohlenstoffatomen, Fluor, Chlor, Brom, Alkoxycarbonyl mit 1 bis 8 Kohlenstoffatomen in der Alkoxygruppe, Cycloalkyloxycarbonyl mit 3 bis 8 Kohlenstoffatomen in der Cycloalkylgruppe, Aminocarbonyl, Alkylaminocarbonyl mit 1 bis 4 Kohlenstoffatomen in der Alkylgruppe, Dialkylaminocarbonyl mit 1 bis 4 Kohlenstoffatomen in jeder Alkylgruppe, Cycloalkylaminocarbonyl mit 3 bis 7 Kohlenstoffatomen in der Cycloalkylgruppe, Dicycloalkyl-aminocarbonyl mit 3 bis 7 Kohlenstoffatomen in jeder Cycloalkylgruppe, gegebenenfalls durch Alkyl mit 1 bis 3 Kohlenstoffatomen, Fluor, Chlor und/oder Brom substituiertes Phenoxycarbonyl oder für Cyano steht,

m für 1 oder 2 steht
n für 1 oder 2 steht und
p für 1 oder 2 steht, und
$R^5$ außerdem auch für Wasserstoff oder Chlor steht, sofern $R^3$ nicht für Wasserstoff, Chlor, Methyl oder Nitro steht,

dadurch gekennzeichnet, daß man

a) Thiophenol-Derivate der Formel

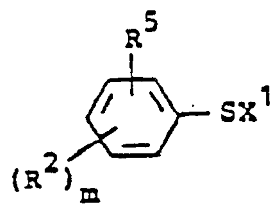

(II)

in welcher
$R^2$, $R^5$ und m die oben angegebene Bedeutung haben und
$X^1$ für Wasserstoff oder ein Alkalimetallatom steht,

mit Halogenmethyl-phenyl-ethern der Formel

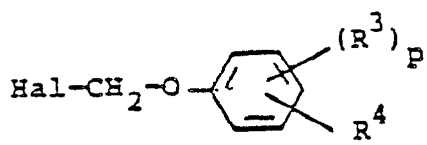

(III)

in welcher
$R^3$, $R^4$ und p die oben angegebene Bedeutung haben und
Hal für Chlor, Brom oder Jod steht,

gegebenenfalls in Gegenwart eines Säureakzeptors sowie gegebenenfalls in Gegenwart eines Verdünnungsmittels umsetzt und anschließend die dabei entstehenden Phenoxymethan-Derivate der Formel

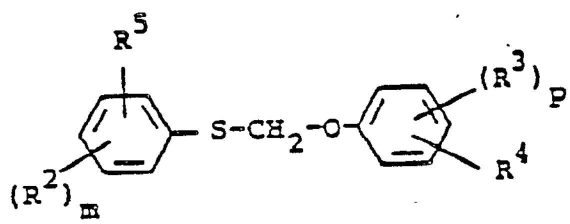

(Ic)

in welcher $R^2$, $R^3$, $R^4$, $R^5$, m und p die oben angegebene Bedeutung haben, mit einem Oxidationsmittel gegebenenfalls in Gegenwart eines Verdünnungsmittels umsetzt, oder

b) Phenol-Derivate der Formel

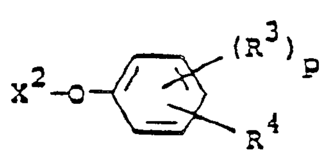

(IV)

in welcher
$R^3$, $R^4$ und p die oben angegebene Bedeutung haben und
$X^2$ für Wasserstoff oder ein Alkalimetallatom steht,

mit Halogenmethyl-phenylsulfiden der Formel

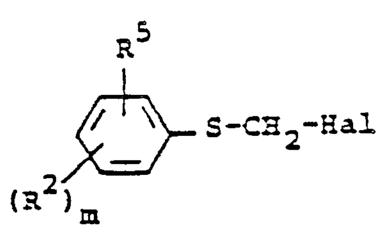

(V)

in welcher

$R^2$, $R^5$ und m die oben angegebene Bedeutung haben und

Hal für Chlor, Brom oder Jod steht,

gegebenenfalls in Gegenwart eines Säureakzeptors sowie gegebenenfalls in Gegenwart eines Verdünnungsmittels umsetzt und anschließend die dabei entstehenden Phenoxymethan-Derivate der Formel

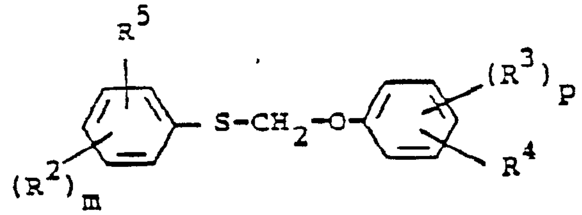

(Ic)

in welcher $R^2$, $R^3$, $R^4$, $R^5$, m und p die oben angegebene Bedeutung haben, mit einem Oxidationsmittel gegebenenfalls in Gegenwart eines Verdünnungsmittels umsetzt, oder

c) Sulfinsäure-Salze der Formel

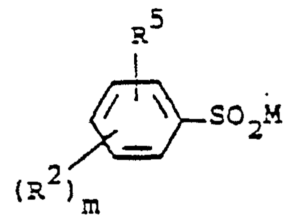

(VI)

in welcher

$R^2$, $R^5$ und m die oben angegebene Bedeutung haben und

M für ein Alkalimetallatom steht,

mit Halogenmethyl-phenyl-ethern der Formel

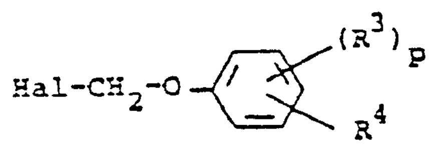

(III)

in welcher

$R^3$, $R^4$ und p die oben angegebene Bedeutung haben und

Hal für Chlor, Brom oder Jod steht,

gegebenenfalls in Gegenwart eines Katalysators sowie gegebenenfalls in Gegenwart eines Verdünnungsmittels umsetzt.

4. Phenoxymethan-Derivate der Formel

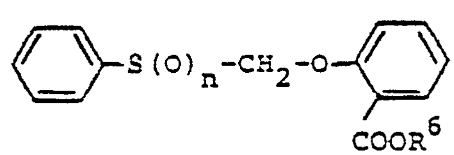

(Id)

in welcher

n für 0, 1 oder 2 steht und

$R^6$ für Wasserstoff, ein Natrium- oder Kalium-Kation, ein Äquivalent eines Magnesium- oder Calcium-Kations, Alkyl mit 2 bis 8 Kohlenstoffatomen, Alkenyl mit 2 bis 8 Kohlenstoffatomen, Alkoxyalkyl mit 1 bis 4 Kohlenstoffatomen in der Alkoxygruppe und 1 bis 4 Kohlenstoffatomen im Alkylteil, Alkylthioalkyl mit 1 bis 4 Kohlenstoffatomen in der Alkylthiogruppe und 1 bis 4 Kohlenstoffatomen im Alkylteil oder für den Rest der Formel $-CH_2-O-(CH_2-CH_2-O)_q$-Alk steht, worin

Alk für Alkyl mit 1 bis 4 Kohlenstoffatomen steht und

q für 0, 1 oder 2 steht.

5. Verfahren zur Herstellung von Phenoxymethan-Derivaten der Formel

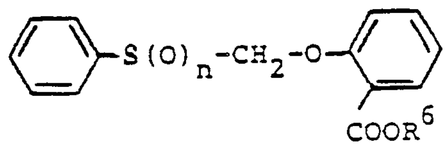

(Id)

in welcher

n für 0, 1 oder 2 steht und

$R^6$ für Wasserstoff, ein Natrium- oder Kalium-Kation, ein Äquivalent eines Magnesium- oder Calcium-Kations, Alkyl mit 2 bis 8 Kohlenstoffatomen, Alkenyl mit 2 bis 8 Kohlenstoffatomen, Alkoxyalkyl mit 1 bis 4 Kohlenstoffatomen in der Alkoxygruppe und 1 bis 4 Kohlenstoffatomen im Alkylteil, Alkylthioalkyl mit 1 bis 4 Kohlenstoffatomen in der Alkylthiogruppe und 1 bis 4 Kohlenstoffatomen im Alkylteil oder für den Rest der Formel $—CH_2—O—(CH_2—CH_2—O)_q$-Alk steht, worin

Alk für Alkyl mit 1 bis 4 Kohlenstoffatomen steht und

q für 0, 1 oder 2 steht,

dadurch gekennzeichnet, daß man

d) Verbindungen der Formel

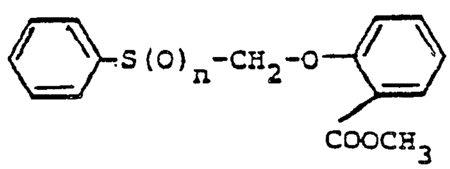

(Ie)

in welcher n die oben angegebene Bedeutung hat, entweder

α) mit Verbindungen der Formel

$$R^7OH \quad (VII)$$

in welcher $R^7$ für Alkyl mit 2 bis 8 Kohlenstoffatomen, Alkenyl mit 2 bis 8 Kohlenstoffatomen, Alkoxyalkyl mit 1 bis 4 Kohlenstoffatomen in der Alkoxygruppe und 1 bis 4 Kohlenstoffatomen im Alkylteil, Alkylthioalkyl mit 1 bis 4 Kohlenstoffatomen in der Alkylthiogruppe und 1 bis 4 Kohlenstoffatomen im Alkylteil oder für den Rest der Formel $—CH_2—O—(CH_2—CH_2—O)_q$-Alk steht, worin

Alk für Alkyl mit 1 bis 4 Kohlenstoffatomen steht und

q für 0, 1 oder 2 steht,

in Gegenwart einer Base sowie gegebenenfalls in Gegenwart eines Verdünnungsmittels umsetzt, oder

β) mit Basen der Formel

$$M^1OH \quad (VIII)$$

in welcher $M^1$ für ein Natrium- oder Kalium-Kation oder für ein Äquivalent eines Magnesium- oder Calcium-Kations steht, in Gegenwart eines Verdünnungsmittels umsetzt und dann mit einer starken Säure ansäuert und gegebenenfalls anschließend erneut mit einer Base der Formel

$$M^1OH \quad (VIII)$$

in welcher $M^1$ die oben angegebene Bedeutung hat, in Gegenwart eines Verdünnungsmittels umsetzt.

## Claims

1. Use of phenoxymethane derivatives of the formula

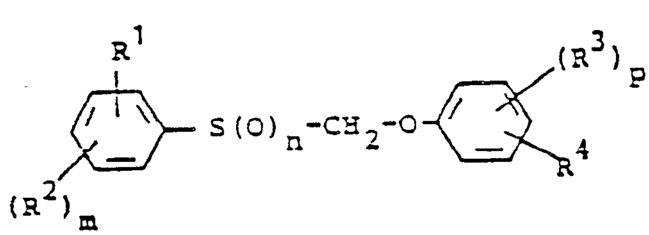

(I)

in which

$R^1$ represents hydrogen, straight-chain or branched alkyl with 1 to 6 carbon atoms, straight-chain or branched halogenoalkyl with 1 to 6 carbon atoms and 1 to 5 halogen atoms, straight-chain or branched alkoxy with 1 to 6 carbon atoms, halogenoalkoxy with 1 to 6 carbon atoms and 1 to 5 halogen atoms, alkoxycarbonyl with 1 to 10 carbon atoms in the alkoxy group, cycloalkyloxycarbonyl with 3 to 8 carbon atoms in the cycloalkyl group, fluorine, chlorine, bromine, iodine or nitro,

$R^2$ represents hydrogen, straight-chain or branched alkyl with 1 to 4 carbon atoms, fluorine, chlorine, bromine or iodine,

$R^3$ represents hydrogen, straight-chain or branched alkyl with 1 to 6 carbon atoms, straight-chain or branched alkenyl with 3 to 6 carbon atoms, straight-chain or branched alkinyl with 3 to 6 carbon atoms, phenyl which is optionally substituted by alkyl with 1 to 4 carbon atoms, fluorine, chlorine, bromine and/or iodine, benzyl which is optionally substituted by alkyl with 1 to 4 carbon atoms, fluorine, chlorine, bromine and/or iodine, alkoxy with 1 to 6 carbon atoms, phenoxy, halogenoalkyl with 1 to 6 carbon atoms and 1 to 5 halogen atoms, halogenoalkoxy with 1 to 6 carbon atoms and 1 to 5 halogen atoms, alkylthio with 1 to 6 carbon atoms, halogenoalkylthio with 1 to 6 carbon atoms and 1 to 5 halogen atoms, alkylsulphonyl with 1 to 6 carbon atoms, phenylthio, phenylsulphonyl, nitro, fluorine, chlorine, bromine or iodine,

$R^4$ represents hydrogen, straight-chain or branched alkyl with 1 to 6 carbon atoms, fluorine, chlorine, bromine, iodine, cycloalkyloxycarbonyl with up to 10 carbon atoms in the cycloalkyl group, aminocarbonyl, alkylaminocarbonyl with 1 to 6 carbon atoms in the alkyl group, dialkylaminocarbonyl with 1 to 6 carbon atoms in each alkyl group, cycloalkylaminocarbonyl with 3 to 8 carbon atoms in the cycloalkyl group, di-cycloalkylaminocarbonyl with 3 to 8 carbon atoms in each cycloalkyl group, phenoxycarbonyl which is optionally substituted by alkyl with 1 to 4 carbon atoms, fluorine, chlorine, bromine and/or iodine, cyano, or the radical of the formula $-COOR$, wherein

R represents hydrogen, a sodium or potassium cation, one equivalent of a magnesium or calcium cation, alkyl with 1 to 10 carbon atoms, alkenyl with 2 to 10 carbon atoms, alkoxyalkyl with 1 to 6 carbon atoms in the alkoxy group and 1 to 6 carbon atoms in the alkyl part, alkylthioalkyl with 1 to 6 carbon atoms in the alkylthio group and 1 to 6 carbon atoms in the alkyl part, or the radical of the formula $-CH_2-O-(CH_2-CH_2-O)_q$-Alk, wherein

Alk represents alkyl with 1 to 6 carbon atoms and

q represents 0, 1 or 2,

m represents 1 or 2,

n represents 0, 1 or 2 and

p represents 1 or 2,

for regulating plant growth.

2. Phenoxymethane derivatives of the formula

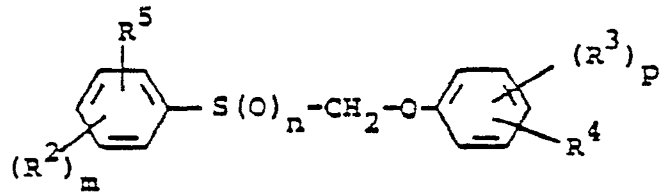

(Ib)

in which

$R^5$ represents straight-chain or branched alkyl with 2 to 4 carbon atoms, straight-chain or branched alkoxy with 1 to 4 carbon atoms, halogenoalkyl with 1 to 3 carbon atoms and 1 to 5 fluorine, chlorine and/or bromine atoms, halogenoalkoxy with 1 to 3 carbon atoms and 1 to 5 fluorine, chlorine and/or bromine atoms, alkoxycarbonyl with 1 to 4 carbon atoms in the alkoxy group, cycloalkyloxycarbonyl with 3 to 6 carbon atoms in the cycloalkyl group, fluorine, bromine or nitro,

$R^2$ represents hydrogen, straight-chain or branched alkyl with 1 to 4 carbon atoms, fluorine, chlorine or bromine,

$R^3$ represents hydrogen, straight-chain or branched alkyl with 1 to 4 carbon atoms, straight-chain or branched alkenyl with 3 to 5 carbon atoms, straight-chain or branched alkinyl with 3 to 5 carbon atoms, phenyl which is optionally substituted by alkyl with 1 to 3 carbon atoms, fluorine, chlorine and/or bromine, benzyl which is optionally substituted by alkyl with 1 to 3 carbon atoms, fluorine, chlorine and/or bromine, alkoxy with 1 to 4 carbon atoms, phenoxy, halogenoalkyl with 1 to 3 carbon atoms and 1 to 5 fluorine, chlorine and/or bromine atoms, halogenoalkoxy with 1 to 3 carbon atoms and 1 to 5 fluorine, chlorine and/or bromine atoms, alkylthio with 1 to 4 carbon atoms, halogenoalkylthio with 1 to 3 carbon atoms and 1 to 5 fluorine, chlorine and/or bromine atoms, alkylsulphonyl with 1 to 4 carbon atoms, phenylthio, phenylsulphonyl, nitro, fluorine, chlorine or bromine,

$R^4$ represents hydrogen, straight-chain or branched alkyl with 1 to 4 carbon atoms, fluorine, chlorine, bromine, alkoxycarbonyl with 1 to 8 carbon atoms in the alkoxy group, cycloalkyloxycarbonyl with 3 to 8 carbon atoms in the cycloalkyl group, aminocarbonyl, alkylaminocarbonyl with 1 to 4 carbon atoms in the

alkyl group, dialkylaminocarbonyl with 1 to 4 carbon atoms in each alkyl group, cycloalkylaminocarbonyl with 3 to 7 carbon atoms in the cycloalkyl group, dicycloalkyl-aminocarbonyl with 3 to 7 carbon atoms in each cycloalkyl group, phenoxycarbonyl which is optionally substituted by alkyl with 1 to 3 carbon atoms, fluorine, chlorine and/or bromine, or cyano,

m represents 1 or 2,

n represents 1 or 2 and

p represents 1 or 2 and

$R^5$ also represents hydrogen or chlorine unless $R^3$ represents hydrogen, chlorine, methyl or nitro.

3. Process for the preparation of phenoxymethane derivatives of the formula

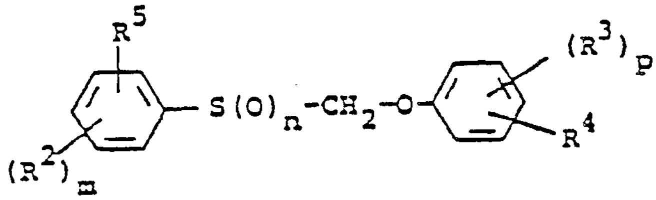

(Ib)

in which

$R^5$ represents straight-chain or branched alkyl with 2 to 4 carbon atoms, straight-chain or branched alkoxy with 1 to 4 carbon atoms, halogenoalkyl with 1 to 3 carbon atoms and 1 to 5 fluorine, chlorine and/or bromine atoms, halogenoalkoxy with 1 to 3 carbon atoms and 1 to 5 fluorine, chlorine and/or bromine atoms, alkoxycarbonyl with 1 to 4 carbon atoms in the alkoxy group, cycloalkyloxycarbonyl with 3 to 6 carbon atoms in the cycloalkyl group, fluorine, bromine or nitro,

$R^2$ represents hydrogen, straight-chain or branched alkyl with 1 to 4 carbon atoms, fluorine, chlorine or bromine,

$R^3$ represents hydrogen, straight-chain or branched alkyl with 1 to 4 carbon atoms, straight-chain or branched alkenyl with 3 to 5 carbon atoms, straight-chain or branched alkinyl with 3 to 5 carbon atoms, phenyl which is optionally substituted by alkyl with 1 to 3 carbon atoms, fluorine, chlorine and/or bromine, benzyl which is optionally substituted by alkyl with 1 to 3 carbon atoms, fluorine, chlorine and/or bromine, alkoxy with 1 to 4 carbon atoms, phenoxy, halogenoalkyl with 1 to 3 carbon atoms and 1 to 5 fluorine, chlorine and/or bromine atoms, halogenoalkoxy with 1 to 3 carbon atoms and 1 to 5 fluorine, chlorine and/or bromine atoms, alkylthio with 1 to 4 carbon atoms, halogenoalkylthio with 1 to 3 carbon atoms and 1 to 5 fluorine, chlorine and/or bromine atoms, alkylsulphonyl with 1 to 4 carbon atoms, phenylthio, phenylsulphonyl, nitro, fluorine, chlorine or bromine,

$R^4$ represents hydrogen, straight-chain or branched alkyl with 1 to 4 carbon atoms, fluorine, chlorine, bromine, alkoxycarbonyl with 1 to 8 carbon atoms in the alkoxy group, cycloalkyloxycarbonyl with 3 to 8 carbon atoms in the cycloalkyl group, aminocarbonyl, alkylaminocarbonyl with 1 to 4 carbon atoms in the alkyl group, dialkylaminocarbonyl with 1 to 4 carbon atoms in each alkyl group, cycloalkylaminocarbonyl with 3 to 7 carbon atoms in the cycloalkyl group, dicycloalkyl-aminocarbonyl with 3 to 7 carbon atoms in each cycloalkyl group, phenoxycarbonyl which is optionally substituted by alkyl with 1 to 3 carbon atoms, fluorine, chlorine and/or bromine, or cyano,

m represents 1 or 2,

n represents 1 or 2 and

p represents 1 or 2 and

$R^5$ also represents hydrogen or chlorine unless $R^3$ represents hydrogen, chlorine, methyl or nitro, characterised in that

a) thiophenol derivatives of the formula

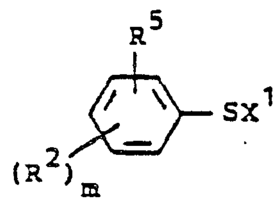

(II)

in which

$R^2$, $R^5$ and m have the abovementioned meaning and

$X^1$ represents hydrogen or an alkali metal atom,

are reacted with halogenomethyl phenyl ethers of the formula

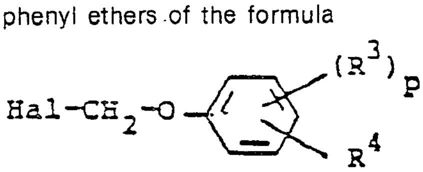

(III)

in which

$R^3$, $R^4$ and p have the abovementioned meaning and

Hal represents chlorine, bromine or iodine,

if appropriate in the presence of an acid acceptor and if appropriate in the presence of a diluent and then the phenoxymethane derivatives thereby formed of the formula

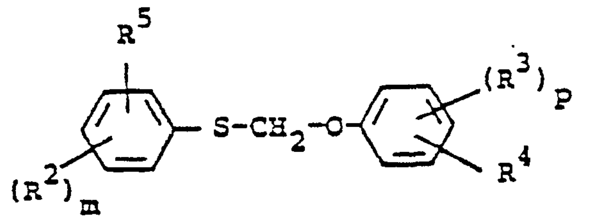

(Ic)

in which $R^2$, $R^3$, $R^4$, $R^5$, m and p have the abovementioned meaning, are reacted with an oxidising agent, if appropriate in the presence of a diluent, or

b) phenol derivatives of the formula

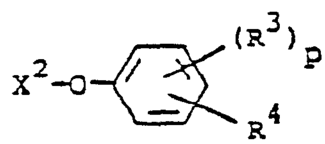

(IV)

in which

$R^3$, $R^4$ and p have the abovementioned meaning and

$X^2$ represents hydrogen or an alkali metal atom,

are reacted with halogenomethyl phenyl sulphides of the formula

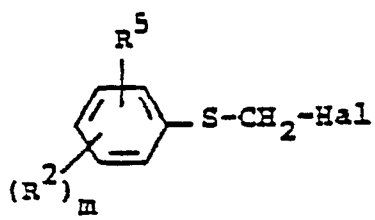

(V)

in which

$R^2$, $R^5$ and m have the abovementioned meaning and

Hal represents chlorine, bromine or iodine,

if appropriate in the presence of an acid acceptor and if appropriate in the presence of a diluent and then the phenoxymethane derivatives thereby formed, of the formula

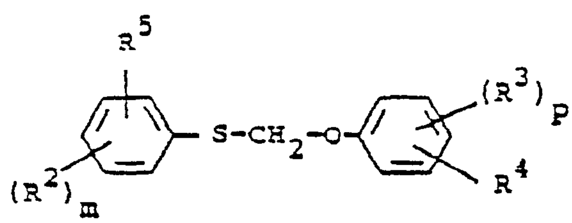

(Ic)

in which $R^2$, $R^3$, $R^4$, $R^5$, m and p have the abovementioned meaning are reacted with an oxidising agent, if appropriate in the presence of a diluent, or

c) sulphinic acid salts of the formula

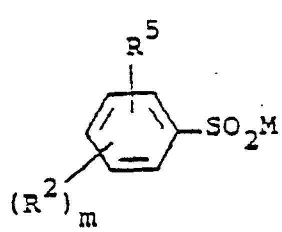

(VI)

in which

R², R⁵ and m have the abovementioned meaning and

M represents an alkali metal atom,

are reacted with halogenomethyl phenyl ethers of the formula

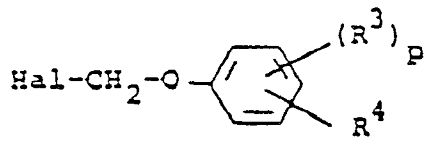

(III)

in which

$R^3$, $R^4$ and p have the abovementioned meaning and

Hal represents chlorine, bromine or iodine,

if appropriate in the presence of a catalyst and if appropriate in the presence of a diluent.

4. Phenoxymethane derivatives of the formula

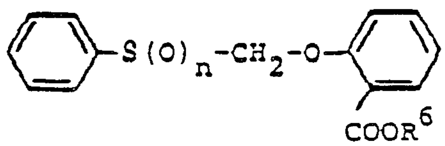

(Id)

in which

n represents 0, 1 or 2 and

$R^6$ represents hydrogen, a sodium or potassium cation, one equivalent of a magnesium or calcium cation, alkyl with 2 to 8 carbon atoms, alkenyl with 2 to 8 carbon atoms, alkoxyalkyl with 1 to 4 carbon atoms in the alkoxy group and 1 to 4 carbon atoms in the alkyl part, alkylthioalkyl with 1 to 4 carbon atoms in the alkylthio group and 1 to 4 carbon atoms in the alkyl part or the radical of the formula $-CH_2-O-(CH_2-CH_2-O)_q$-Alk, wherein

Alk represents alkyl with 1 to 4 carbon atoms and

q represents 0, 1 or 2.

5. Process for the preparation of phenoxymethane derivatives of the formula

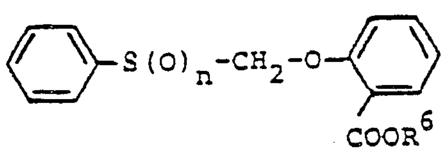

(Id)

in which

n represents 0, 1 or 2 and

$R^6$ represents hydrogen, a sodium or potassium cation, one equivalent of a magnesium or calcium cation, alkyl with 2 to 8 carbon atoms, alkenyl with 2 to 8 carbon atoms, alkoxyalkyl with 1 to 4 carbon atoms in the alkoxy group and 1 to 4 carbon atoms in the alkyl part, alkylthioalkyl with 1 to 4 carbon atoms in the alkylthio group and 1 to 4 carbon atoms in the alkyl part or the radical of the formula $-CH_2-O-(CH_2-CH_2-O)_q$-Alk, wherein

Alk represents alkyl with 1 to 4 carbon atoms and

q represents 0, 1 or 2,

characterised in that

d) compounds of the formula

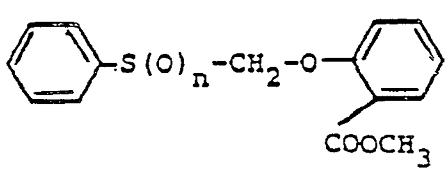

(Ie)

in which n has the abovementioned meaning, either

α) are reacted with compounds of the formula

$R^7OH$ (VII)

in which $R^7$ represents alkyl with 2 to 8 carbon atoms, alkenyl with 2 to 8 carbon atoms, alkoxyalkyl with 1 to 4 carbon atoms in the alkoxy group and 1 to 4 carbon atoms in the alkyl part, alkylthioalkyl with 1 to 4 carbon atoms in the alkylthio group and 1 to 4 carbon atoms in the alkyl part or the radical of the formula $-CH_2-O-(CH_2-CH_2-O)_q$-Alk, wherein

Alk represents alkyl with 1 to 4 carbon atoms and

q represents 0, 1 or 2,

in the presence of a base and if appropriate in the presence of a diluent, or

β) are reacted with bases of the formula

$$M^1OH \quad (VIII)$$

in which $M^1$ represents a sodium or potassium cation or one equivalent of a magnesium or calcium cation, in the presence of a diluent and the products are then acidified with a strong acid and, if appropriate, subsequently reacted again with a base of the formula

$$M^1OH \quad (VIII)$$

in which $M^1$ has the abovementioned meaning, in the presence of a diluent.

**Revendications**

1. Utilisation de dérivés de phénoxyméthane de formule :

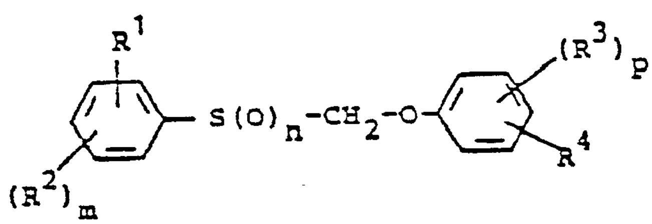

(I)

dans laquelle

$R^1$ représente un atome d'hydrogène, un groupe alkyle à chaîne droite ou ramifiée contenant 1 à 6 atomes de carbone, un groupe halogénalkyle à chaîne droite ou ramifiée contenant 1 à 6 atomes de carbone et 1 à 5 atomes d'halogènes, un groupe alcoxy à chaîne droite ou ramifiée contenant 1 à 6 atomes de carbone, un groupe halogénalcoxy contenant 1 à 6 atomes de carbone et 1 à 5 atomes d'halogènes, un groupe alcoxycarbonyle contenant 1 à 10 atomes de carbone dans le groupe alcoxy, un groupe cycloalkyloxycarbonyle contenant 3 à 8 atomes de carbone dans le groupe cycloalkyle, un atome de fluor, un atome de chlore, un atome de brome, un atome d'iode ou un groupe nitro,

$R^2$ représente un atome d'hydrogène, un groupe alkyle à chaîne droite ou ramifiée contenant 1 à 4 atomes de carbone, un atome de fluor, un atome de chlore, un atome de brome ou un atome d'iode,

$R^3$ représente un atome d'hydrogène, un groupe alkyle à chaîne droite ou ramifiée contenant 1 à 6 atomes de carbone, un groupe alcényle à chaîne droite ou ramifiée contenant 3 à 6 atomes de carbone, un groupe alcynyle à chaîne droite ou ramifiée contenant 3 à 6 atomes de carbone, un groupe phényle éventuellement substitué par un groupe alkyle contenant 1 à 4 atomes de carbone, par un atome de fluor, par un atome de chlore, par un atome de brome et/ou par un atome d'iode, un groupe benzyle éventuellement substitué par un groupe alkyle contenant 1 à 4 atomes de carbone, par un atome de fluor, par un atome de chlore, par un atome de brome et/ou par un atome d'iode, un groupe alcoxy contenant 1 à 6 atomes de carbone, un groupe phénoxy, un groupe halogénalkyle contenant 1 à 6 atomes de carbone et 1 à 5 atomes d'halogènes, un groupe halogénalcoxy contenant 1 à 6 atomes de carbone et 1 à 5 atomes d'halogènes, un groupe alkylthio contenant 1 à 6 atomes de carbone, un groupe halogénalkylthio contenant 1 à 6 atomes de carbone et 1 à 5 atomes d'halogènes, un groupe alkylsulfonyle contenant 1 à 6 atomes de carbone, un groupe phénylthio, un groupe phénylsulfonyle, un groupe nitro, un atome de fluor, un atome de chlore, un atome de brome ou un atome d'iode,

$R^4$ représente un atome d'hydrogène, un groupe alkyle à chaîne droite ou ramifiée contenant 1 à 6 atomes de carbone, un atome de fluor, un atome de chlore, un atome de brome, un atome d'iode, un groupe cycloalkyloxycarbonyle contenant jusqu'à 10 atomes de carbone dans le groupe cycloalkyle, un groupe aminocarbonyle, un groupe alkylaminocarbonyle contenant 1 à 6 atomes de carbone dans le groupe alkyle, un groupe dialkylaminocarbonyle contenant 1 à 6 atomes de carbone dans chaque groupe alkyle, un groupe cycloalkylaminocarbonyle contenant 3 à 8 atomes de carbone dans le groupe cycloalkyle, un groupe dicycloalkylaminocarbonyle contenant 3 à 8 atomes de carbone dans chaque groupe cycloalkyle, un groupe phénoxycarbonyle éventuellement substitué par un groupe alkyle contenant 1 à 4 atomes de carbone, par un atome de fluor, par un atome de chlore, par un atome de

brome et/ou par un atome d'iode, un groupe cyano ou le radical de formule —COOR, où

R représente un atome d'hydrogène, un cation sodium ou potassium, un équivalent d'un cation magnésium ou calcium, un groupe alkyle contenant 1 à 10 atomes de carbone, un groupe alcényle contenant 2 à 10 atomes de carbone, un groupe alcoxyalkyle contenant 1 à 6 atomes de carbone dans le groupe alcoxy et 1 à 6 atomes de carbone dans la fraction alkyle, un groupe alkylthioalkyle contenant 1 à 6 atomes de carbone dans le groupe alkylthio et 1 à 6 atomes de carbone dans la fraction alkyle ou le radical de formule $—CH_2—O—(CH_2—CH_2—O)_q$-Alk, où

Alk représente un groupe alkyle contenant 1 à 6 atomes de carbone, et

q représente 0, 1 ou 2,

m représente 1 ou 2,

n représente 0, 1 ou 2, et

p représente 1 ou 2,

pour la régulation de la croissance des plantes.

2. Dérivés de phénoxyméthane de formule :

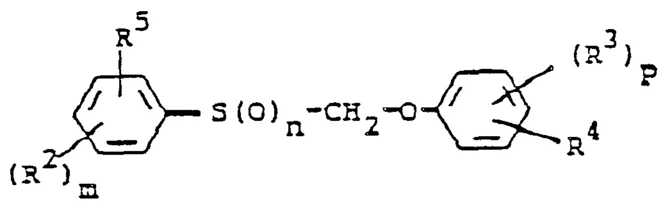

(Ib)

dans laquelle

$R^5$ représente un groupe alkyle à chaîne droite ou ramifiée contenant 2 à 4 atomes de carbone, un groupe alcoxy à chaîne droite ou ramifiée contenant 1 à 4 atomes de carbone, un groupe halogénalkyle contenant 1 à 3 atomes de carbone et 1 à 5 atomes de fluor, de chlore et/ou de brome, un groupe halogénalcoxy contenant 1 à 3 atomes de carbone et 1 à 5 atomes de fluor, de chlore et/ou de brome, un groupe alcoxycarbonyle contenant 1 à 4 atomes de carbone dans le groupe alcoxy, un groupe cycloalkyloxycarbonyle contenant 3 à 6 atomes de carbone dans le groupe cycloalkyle, un atome de fluor, un atome de brome ou un groupe nitro,

$R^2$ représente un atome d'hydrogène, un groupe alkyle à chaîne droite ou ramifiée contenant 1 à 4 atomes de carbone, un atome de fluor, un atome de chlore ou un atome de brome,

$R^3$ représente un atome d'hydrogène, un groupe alkyle à chaîne droite ou ramifiée contenant 1 à 4 atomes de carbone, un groupe alcényle à chaîne droite ou ramifiée contenant 3 à 5 atomes de carbone, un groupe alcynyle à chaîne droite ou ramifiée contenant 3 à 5 atomes de carbone, un groupe phényle éventuellement substitué par un groupe alkyle contenant 1 à 3 atomes de carbone, par un atome de fluor, par un atome de chlore et/ou par un atome de brome, un groupe benzyle éventuellement substitué par un groupe alkyle contenant 1 à 3 atomes de carbone, par un atome de fluor, par un atome de chlore et/ou par un atome de brome, un groupe alcoxy contenant 1 à 4 atomes de carbone, un groupe phénoxy, un groupe halogénalkyle contenant 1 à 3 atomes de carbone et 1 à 5 atomes de fluor, de chlore et/ou de brome, un groupe halogénalcoxy contenant 1 à 3 atomes de carbone et 1 à 5 atomes de fluor, de chlore et/ou de brome, un groupe alkylthio contenant 1 à 4 atomes de carbone, un groupe halogénalkylthio contenant 1 à 3 atomes de carbone et 1 à 5 atomes de fluor, de chlore et/ou de brome, un groupe alkylsulfonyle contenant 1 à 4 atomes de carbone, un groupe phénylthio, un groupe phénylsulfonyle, un groupe nitro, un atome de fluor, un atome de chlore ou un atome de brome,

$R^4$ représente un atome d'hydrogène, un groupe alkyle à chaîne droite ou ramifiée contenant 1 à 4 atomes de carbone, un atome de fluor, un atome de chlore, un atome de brome, un groupe alcoxycarbonyle contenant 1 à 8 atomes de carbone dans le groupe alcoxy, un groupe cycloalkyloxycarbonyle contenant 3 à 8 atomes de carbone dans le groupe cycloalkyle, un groupe aminocarbonyle, un groupe alkylaminocarbonyle contenant 1 à 4 atomes de carbone dans le groupe alkyle, un groupe dialkylaminocarbonyle contenant 1 à 4 atomes de carbone dans chaque groupe alkyle, un groupe cycloalkylaminocarbonyle contenant 3 à 7 atomes de carbone dans le groupe cycloalkyle, un groupe dicycloalkylaminocarbonyle contenant 3 à 7 atomes de carbone dans chaque groupe cycloalkyle, un groupe phénoxycarbonyle éventuellement substitué par un groupe alkyle contenant 1 à 3 atomes de carbone, par un atome de fluor, par un atome de chlore et/ou par un atome de brome, ou un groupe cyano,

m représente 1 ou 2,

n représente 1 ou 2,

p représente 1 ou 2, et

$R^5$ représente en outre un atome d'hydrogène ou un atome de chlore, pour autant que $R^3$ ne représente pas un atome d'hydrogène, un atome de chlore, un groupe méthyle ou un groupe nitro.

3. Procédé de préparation de dérivés de phénoxyméthane de formule :

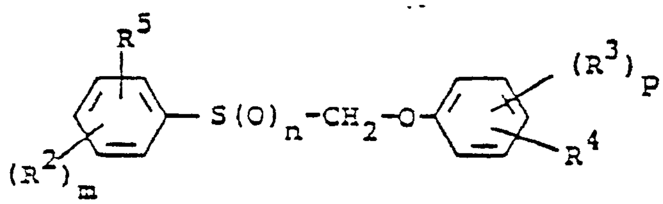 (Ib)

dans laquelle

$R^5$ représente un groupe alkyle à chaîne droite ou ramifiée contenant 2 à 4 atomes de carbone, un groupe alcoxy à chaîne droite ou ramifiée contenant 1 à 4 atomes de carbone, un groupe halogénalkyle contenant 1 à 3 atomes de carbone et 1 à 5 atomes de fluor, de chlore et/ou de brome, un groupe halogénalcoxy contenant 1 à 3 atomes de carbone et 1 à 5 atomes de fluor, de chlore et/ou de brome, un groupe alcoxycarbonyle contenant 1 à 4 atomes de carbone dans le groupe alcoxy, un groupe cycloalkyloxycarbonyle contenant 3 à 6 atomes de carbone dans le groupe cycloalkyle, un atome de fluor, un atome de brome ou un groupe nitro,

$R^2$ représente un atome d'hydrogène, un groupe alkyle à chaîne droite ou ramifiée contenant 1 à 4 atomes de carbone, un atome de fluor, un atome de chlore ou un atome de brome,

$R^3$ représente un atome d'hydrogène, un groupe alkyle à chaîne droite ou ramifiée contenant 1 à 4 atomes de carbone, un groupe alcényle à chaîne droite ou ramifiée contenant 3 à 5 atomes de carbone, un groupe alcynyle à chaîne droite ou ramifiée contenant 3 à 5 atomes de carbone, un groupe phényle éventuellement substitué par un groupe alkyle contenant 1 à 3 atomes de carbone, par un atome de fluor, par un atome de chlore et/ou par un atome de brome, un groupe benzyle éventuellement substitué par un groupe alkyle contenant 1 à 3 atomes de carbone, par un atome de fluor, par un atome de chlore et/ou par un atome de brome, un groupe alcoxy contenant 1 à 4 atomes de carbone, un groupe phénoxy, un groupe halogénalkyle contenant 1 à 3 atomes de carbone et 1 à 5 atomes de fluor, de chlore et/ou de brome, un groupe halogénalcoxy contenant 1 à 3 atomes de carbone et 1 à 5 atomes de fluor, de chlore et/ou de brome, un groupe alkylthio contenant 1 à 4 atomes de carbone, un groupe halogénalkylthio contenant 1 à 3 atomes de carbone et 1 à 5 atomes de fluor, de chlore et/ou de brome, un groupe alkylsulfonyle contenant 1 à 4 atomes de carbone, un groupe phénylthio, un groupe phénylsulfonyle, un groupe nitro, un atome de fluor, un atome de chlore ou un atome de brome,

$R^4$ représente un atome d'hydrogène, un groupe alkyle à chaîne droite ou ramifiée contenant 1 à 4 atomes de carbone, un atome de fluor, un atome de chlore, un atome de brome, un groupe alcoxycarbonyle contenant 1 à 8 atomes de carbone dans le groupe alcoxy, un groupe cycloalkyloxycarbonyle contenant 3 à 8 atomes de carbone dans le groupe cycloalkyle, un groupe aminocarbonyle, un groupe alkylaminocarbonyle contenant 1 à 4 atomes de carbone dans le groupe alkyle, un groupe dialkylaminocarbonyle contenant 1 à 4 atomes de carbone dans chaque groupe alkyle, un groupe cycloalkylaminocarbonyle contenant 3 à 7 atomes de carbone dans le groupe cycloalkyle, un groupe dicycloalkylaminocarbonyle contenant 3 à 7 atomes de carbone dans chaque groupe cycloalkyle, un groupe phénoxycarbonyle éventuellement substitué par un groupe alkyle contenant 1 à 3 atomes de carbone, par un atome de fluor, par un atome de chlore et/ou par un atome de brome, ou encore un groupe cyano,

m représente 1 ou 2,

n représente 1 ou 2,

p représente 1 ou 2, et

$R^5$ représente, en outre, un atome d'hydrogène ou un atome de chlore, pour autant que $R^3$ ne représente pas un atome d'hydrogène, un atome de chlore, un groupe méthyle ou un groupe nitro,

caractérisé en ce que :

a) on fait réagir des dérivés de thiophénol de formule :

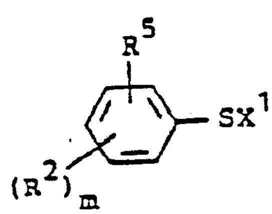 (II)

dans laquelle

$R^2$, $R^5$ et m ont les significations indiquées ci-dessus, et

$X^1$ représente un atome d'hydrogène ou un atome d'un métal alcalin,

avec des éthers halogénométhyl-phényliques de formule :

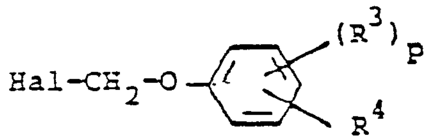 (III)

dans laquelle

$R^3$, $R^4$ et p ont les significations indiquées ci-dessus, et

Hal représente un atome de chlore, un atome de brome ou un atome d'iode,

éventuellement en présence d'un accepteur d'acide, ainsi qu'éventuellement en présence d'un diluant, puis on fait réagir les dérivés de phénoxyméthane ainsi obtenus de formule :

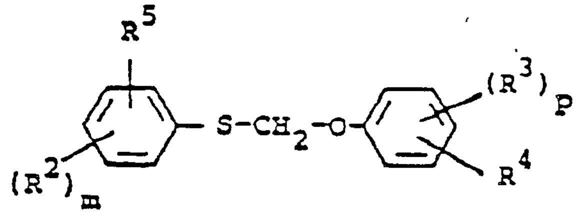 (Ic)

dans laquelle $R^2$, $R^3$, $R^4$, $R^5$, m et p ont les significations indiquées ci-dessus, avec un agent oxydant éventuellement en présence d'un diluant, ou

b) on fait réagir des dérivés de phénol de formule :

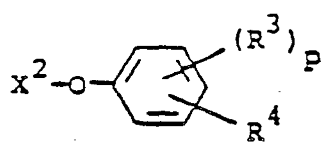 (IV)

dans laquelle

$R^3$, $R^4$ et p ont les significations indiquées ci-dessus, et

$X^2$ représente un atome d'hydrogène ou un atome de métal alcalin,

avec des sulfures d'halogénométhyl-phényle de formule :

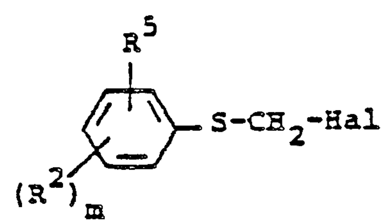 (V)

dans laquelle

$R^2$, $R^5$ et m ont les significations indiquées ci-dessus, et

Hal représente un atome de chlore, un atome de brome ou un atome d'iode,

éventuellement en présence d'un accepteur d'acide, ainsi qu'éventuellement en présence d'un diluant, puis on fait réagir les dérivés de phénoxyméthane ainsi formés de formule :

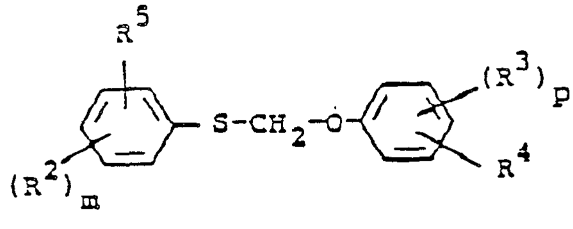 (Ic)

dans laquelle $R^2$, $R^3$, $R^4$, $R^5$, m et p ont les significations indiquées ci-dessus, avec un agent oxydant éventuellement en présence d'un diluant, ou

c) on fait réagir des sels de l'acide sulfinique de formule :

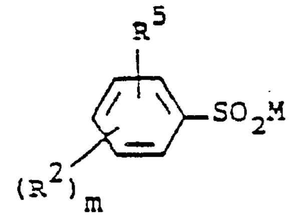

(VI)

dans laquelle
$R^2$, $R^5$ et m ont les significations indiquées ci-dessus, et
M représente un atome de métal alcalin,
avec des éthers halogénométhyl-phényliques de formule :

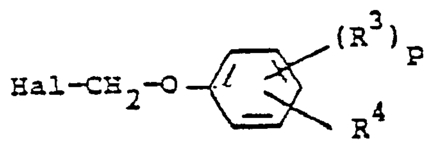

(III)

dans laquelle
$R^3$, $R^4$ et p ont les significations indiquées ci-dessus, et
Hal représente un atome de chlore, un atome de brome ou un atome d'iode,
éventuellement en présence d'un catalyseur, ainsi qu'éventuellement en présence d'un diluant.

4. Dérivés de phénoxyméthane de formule :

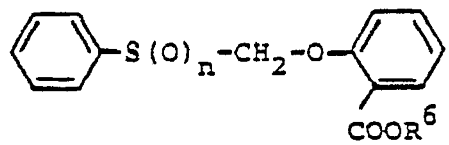

(Id)

dans laquelle
n représente 0, 1 ou 2, et
$R^6$ représente un atome d'hydrogène, un cation sodium ou potassium, un équivalent d'un cation magnésium ou calcium, un groupe alkyle contenant 2 à 8 atomes de carbone, un groupe alcényle contenant 2 à 8 atomes de carbone, un groupe alcoxyalkyle contenant 1 à 4 atomes de carbone dans le groupe alcoxy et 1 à 4 atomes de carbone dans la fraction alkyle, un groupe alkylthioalkyle contenant 1 à 4 atomes de carbone dans le groupe alkylthio et 1 à 4 atomes de carbone dans la fraction alkyle ou le radical de formule $-CH_2-O-(CH_2-CH_2-O)_q$-Alk, où
Alk représente un groupe alkyle contenant 1 à 4 atomes de carbone et
q représente 0, 1 ou 2.

5. Procédé de préparation de dérivés de phénoxyméthane de formule :

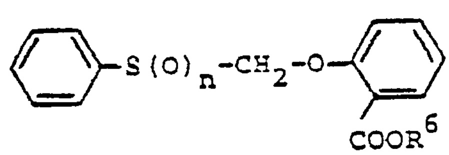

(Id)

dans laquelle
n représente 0, 1 ou 2, et
$R^6$ représente un atome d'hydrogène, un cation sodium ou potassium, un équivalent d'un cation magnésium ou calcium, un groupe alkyle contenant 2 à 8 atomes de carbone, un groupe alcényle contenant 2 à 8 atomes de carbone, un groupe alcoxyalkyle contenant 1 à 4 atomes de carbone dans le groupe alcoxy et 1 à 4 atomes de carbone dans la fraction alkyle, un groupe alkylthioalkyle contenant 1 à 4 atomes de carbone dans le groupe alkylthio et 1 à 4 atomes de carbone dans la fraction alkyle ou le radical de formule $-CH_2-O-(CH_2-CH_2-O)_q$-Alk, où
Alk représente un groupe alkyle contenant 1 à 4 atomes de carbone, et
q représente 0, 1 ou 2,
caractérisé en ce que :
d) on fait réagir des composés de formule :

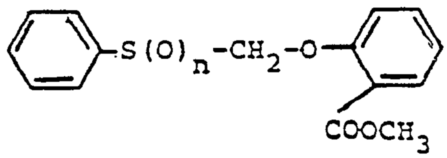

(Ie)

dans laquelle n a la signification indiquée ci-dessus,

α) avec des composés de formule :

$$R^7OH \quad (VII)$$

dans laquelle $R^7$ représente un groupe alkyle contenant 2 à 8 atomes de carbone, un groupe alcényle contenant 2 à 8 atomes de carbone, un groupe alcényle contenant 2 à 8 atomes de carbone, un groupe alcoxyalkyle contenant 1 à 4 atomes de carbone dans le groupe alcoxy et 1 à 4 atomes de carbone dans la fraction alkyle, un groupe alkylthioalkyle contenant 1 à 4 atomes de carbone dans le groupe alkylthio et 1 à 4 atomes de carbone dans la fraction alkyle ou le radical de formule $-CH_2-O-(CH_2-CH_2-O)_q$-Alk où

Alk représente un groupe alkyle contenant 1 à 4 atomes de carbone, et

q représente 0, 1 ou 2,

en présence d'une base, ainsi qu'éventuellement en présence d'un diluant, ou

β) avec des bases de formule :

$$M^1OH \quad (VIII)$$

dans laquelle $M^1$ représente un cation sodium ou potassium ou un équivalent d'un cation magnésium ou calcium, en présence d'un diluant, puis on acidifie avec un acide fort et ensuite, on fait éventuellement à nouveau réagir avec une base de formule :

$$M^1OH \quad (VIII)$$

dans laquelle $M^1$ a la signification indiquée ci-dessus, en présence d'un diluant.